# EUROPEAN PATENT APPLICATION

(11) **EP 4 834 129 A1**
(43) Date of publication of application: **04.11.2026**
(21) Application number: 24912993.3
(22) Date of filing: 26.12.2024
(51) Int. Cl.: A24B 15/10, A01H 1/00, A01H 5/00, A01H 6/82, C12N 15/01, C12N 15/29, C12N 15/55, C12N 15/90

(54) **TOBACCO MATERIAL, TOBACCO PRODUCT, AND PLANT OF GENUS NICOTIANA**

(30) Priority: 27.12.2023 JP 2023221347
(71) Applicant: Japan Tobacco Inc., Tokyo 105-6927 (JP)
(72) Inventor: MIKAMI, Masafumi, Tokyo 130-8603 (JP); TAKAKURA, Yoshimitsu, Tokyo 130-8603 (JP); TAKEUCHI, Takanori, Tokyo 130-8603 (JP); UDAGAWA, Hisashi, Tokyo 130-8603 (JP); MAGOME, Hiroshi, Tokyo 130-8603 (JP); OYAMA, Kiyoshi, Tokyo 130-8603 (JP); SATO, Moeko, Tokyo 130-8603 (JP); ZHU, Zheng, Tokyo 130-8603 (JP); MIYATA, Soshiro, Tokyo 130-8603 (JP); AYABE, Hiroki, Tokyo 130-8603 (JP); KATO, Sho, Tokyo 130-8603 (JP)
(74) Representative: Hoffmann Eitle
(86) International application number: PCT/JP2024/046123
(87) International publication number: WO 2025/143107

(57) **Abstract**

To realize a tobacco product with a better smoking taste. A tobacco material comprising a portion of a Nicotiana plant, wherein the Nicotiana plant has a mutation accompanied by functional suppression of an endogenous CAD gene, and the tobacco material contains 0.05 ppm or more of vanillin.

## Description

### TECHNICAL FIELD

The present invention relates to a tobacco material, a tobacco product, and a Nicotiana plant.

### BACKGROUND ART

The smoking taste of tobacco products is a complex quality appealing to the human sensory organs, and it is believed that the smoking taste is based on a balance of various components contained in the leaf tobacco material. Many components contributing to the smoking taste have been studied to improve the smoking taste of tobacco. PTL 1 indicates that a tobacco raw material is processed by industrial methods to produce a tobacco material with an increased content of smoking taste components (e.g. vanillin).

### CITATION LIST

### PATENT LITERATURE

PTL 1: JP 6019216 B2

### SUMMARY OF INVENTION

### PROBLEM TO BE SOLVED BY THE INVENTION

In view of this situation, there are still further investigations to be made into methods with a lower environmental impact, as a means for improving the smoking taste of tobacco products.

One aspect of the present invention aims to realize tobacco products with a better smoking taste.

### SOLUTION TO PROBLEM

In order to solve the problem above, one aspect of the present invention relates to a tobacco material comprising a portion of a Nicotiana plant, wherein the Nicotiana plant has a mutation accompanied by functional suppression of an endogenous cinnamyl alcohol dehydrogenase gene in at least one of: (a) an endogenous cinnamyl alcohol dehydrogenase gene comprising, as a coding region, a polynucleotide encoding a polypeptide having at least 90% sequence identity to the amino acid sequence shown in SEQ ID NO: 1; and (b) an endogenous cinnamyl alcohol dehydrogenase gene comprising, as a coding region, a polynucleotide encoding a polypeptide having at least 90% sequence identity to the amino acid sequence shown in SEQ ID NO: 2, and the tobacco material contains 0.05 ppm or more of vanillin.

Furthermore, in order to solve the problem above, one aspect of the present invention relates to a Nicotiana plant, wherein a mutation inducing functional suppression of an endogenous cinnamyl alcohol dehydrogenase gene is introduced into at least one of: (a) an endogenous cinnamyl alcohol dehydrogenase gene comprising, as a coding region, a polynucleotide encoding a polypeptide having at least 90% sequence identity to the amino acid sequence shown in SEQ ID NO: 1; and (b) an endogenous cinnamyl alcohol dehydrogenase gene comprising, as a coding region, a polynucleotide encoding a polypeptide having at least 90% sequence identity to the amino acid sequence shown in SEQ ID NO: 2, and a vanillin content of a tobacco raw material obtained from the abovementioned Nicotiana plant is higher than a vanillin content of a tobacco raw material obtained from the same amount of a wild-type Nicotiana plant.

### ADVANTAGEOUS EFFECTS OF INVENTION

One aspect of the present invention makes it possible to realize a tobacco product with a better smoking taste.

### BRIEF DESCRIPTION OF DRAWINGS

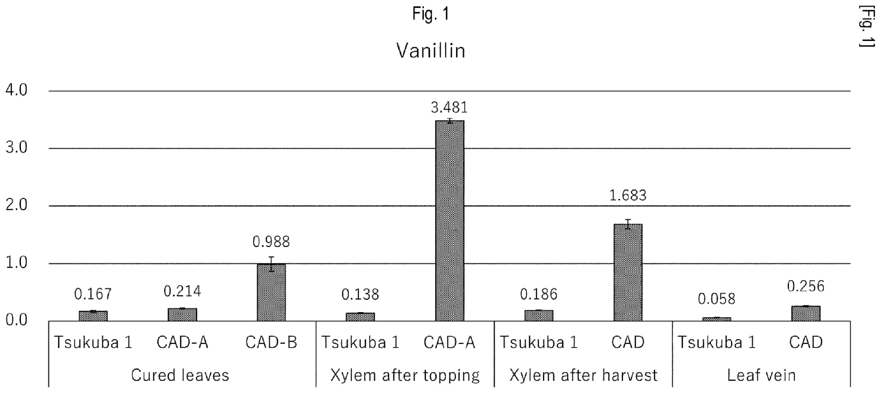

[Fig. 1] shows vanillin contents of Tsukuba 1 (wild-type Nicotiana plant) and CAD mutants.
[Fig. 2] shows the appearance of a CAD-A stem after epidermis removal, and positions of upper and lower regions of the stem.

### EMBODIMENTS OF THE INVENTION

### [Definitions]

A "Nicotiana plant" is a plant of the order Solanales, and Nicotiana plants belong to the family Solanaceae. In addition to taste-material produce such as Nicotiana, there are also Capsicum species, Solanum species, and Solanum lycopersicum species, which are frequently used as spices or food crops, as well as Petunia species and Physalis species, which are often used as ornamental plants, and many of these include useful plants with high commercial crop value. As used herein, the term "Nicotiana plant" encompasses the entire plant (e.g., a mature plant, a seedling, or a seed) as well as tissues thereof (e.g., leaves, stems, flowers, roots, reproductive organs, embryos, and portions thereof, etc.). As used herein, the term "tobacco raw material" encompasses harvested material of the above "Nicotiana plant" and dried products thereof. For example, as used herein, harvested material of the "Nicotiana plant" includes portions such as the leaves (tobacco leaves) and the stem (for example, the remaining stalk) of the Nicotiana plant. As used herein, the term "leaf" alone is intended to refer to the lamina and midrib of a leaf of a Nicotiana plant as an integral unit. When using leaves as a tobacco raw material, the leaf itself may be used as the tobacco raw material, or the lamina and midrib may be separated and either component may be used as the tobacco raw material. Alternatively, the separated lamina and midrib may be combined in any ratio and used as a tobacco raw material. As used herein, the term "dried tobacco material" specifically refers to the dried product of the "tobacco raw material". Any known drying method may be employed, including, for example, flue-curing, hot-air drying, or freeze-drying. The term "tobacco material" includes the dried product of the "tobacco raw material", the "dried tobacco material", and any processed products thereof. Any known processing method may be employed. Details regarding "tobacco material" will be described later. As used in this specification, the definition also encompasses the generation in which a mutation was introduced and subsequent generations obtained by crossing the same.

"Tobacco" mainly refers to Nicotiana plants belonging to the family Solanaceae, and the principal commercial species cultivated on a large scale are Nicotiana tabacum and Nicotiana rustica. In this specification, the term "Nicotiana plant" refers simply to a plant of the genus Nicotiana belonging to the family Solanaceae, with Nicotiana tabacum being a typical example. Nicotiana plants are described in detail in the [3. Nicotiana plant] section.

Cinnamyl alcohol dehydrogenase (also referred to below as "CAD") is an enzyme responsible for the following conversions in the monolignol biosynthetic pathway (Chabannes et al. (2001) Plant J. 28(3):257-270).

(i) conversion of ρ-coumaraldehyde to ρ-coumaryl alcohol
(ii) conversion of caffeic aldehyde to caffeyl alcohol
(iii) conversion of coniferyl aldehyde to coniferyl alcohol
(iv) conversion of 5-hydroxyconiferaldehyde to 5-hydroxyconiferyl alcohol
(v) conversion of sinapaldehyde to sinapyl alcohol
(vi) conversion of cinnamaldehyde to cinnamyl alcohol

The cinnamyl alcohol dehydrogenase gene of a Nicotiana plant is not particularly limited as long as it encodes an enzyme capable of handling any of the conversions (i) to (vi). Vanillin is not produced by the conversions (i) to (vi) in which CAD is directly involved within the monolignol biosynthetic pathway.

For example, in plant species other than Nicotiana, it has been shown in flax (Linum usitatissimum) that vanillin increases in the alkali-treated fraction of heat-dried stems of CAD gene-silenced recombinants. (Preisner et al. (2014) BMC Plant Biology 14:50). In addition, in pineapple (Ananas comosus), it has been shown that alkali treatment of the stem of a natural CAD-gene mutant results in increased vanillin, ferulic acid, and coniferyl aldehyde. (MacKay et al. (1997) PNAS 94(15):8255-8260). In Nicotiana plants, it has been reported that vanillin increases upon alkali treatment of the stems of antisense CAD tobacco recombinants (Yahiaoui et al. (1998) Planta 204:8-15).

As a result of thorough research, the present inventors found that the vanillin content of tobacco raw material obtained from Nicotiana plants having a mutation in a CAD gene is increased compared with tobacco raw material obtained from wild-type Nicotiana plants. Tobacco materials containing such tobacco raw material were then found to have a superior smoking taste.

### [1. Tobacco raw material]

The tobacco material according to an embodiment of the present invention comprises a portion of a Nicotiana plant having a mutation inducing functional suppression of an endogenous cinnamyl alcohol dehydrogenase gene. For example, the tobacco material comprises a tobacco raw material harvested from a Nicotiana plant, and may be obtained by any processing of the tobacco raw material. Nicotiana plants are described in detail in the [3. Nicotiana plant] section.

The tobacco material comprises at least 0.05 ppm of vanillin. This makes it possible to improve the smoking taste of a tobacco product. Vanillin is an organic compound having a vanilloid skeleton (also referred to as a vanillyl group). The vanillin may be either free vanillin or bound vanillin bound to a cell wall component, and is preferably free vanillin. For example, a predetermined content of vanillin represented by CAS No. 121-33-5 is particularly preferred from the standpoint of improving smoking taste. The vanillin content may be 0.05 ppm or more, preferably 5 ppm or more, and more preferably 25 ppm or more. The vanillin content may also be 5000 ppm or less, 2000 ppm or less, 1000 ppm or less, or 250 ppm or less. The smoking taste of a tobacco product can be further improved by using such a tobacco material. The vanillin content may be measured by any method. For example, the vanillin content may be measured by component analysis using conventional GC-MS or LC-MS/MS. In addition, the vanillin content of a sample that has not been subjected to alkali treatment as a pretreatment for an analytical sample may be measured in the component analysis, for example. Alternatively, for the purpose of specifically analyzing the vanillin content, the vanillin content of a sample that has been subjected to alkali treatment and high-temperature/high-pressure treatment, etc. as a pretreatment for an analytical sample may be measured, for example.

The tobacco material according to this embodiment has a higher vanillin content than a tobacco material produced by using a wild-type Nicotiana plant. For example, the vanillin content of the tobacco material according to this embodiment may be at least 1.3 times, at least 9 times, or at least 25 times greater than the vanillin content of a tobacco material produced by using a wild-type Nicotiana plant.

In the present specification, a comparison of the Nicotiana plant according to the embodiment, or a material or product produced by using this plant, and a wild-type Nicotiana plant or a material or product produced by using this plant, shows that both may be produced under substantially the same conditions. In the present specification, "substantially the same conditions" is intended to refer to, for example, conditions that may affect the vanillin content being substantially the same. Conditions that may affect the vanillin content include, for example, plant growth conditions, conditions for obtaining the harvested material, and conditions for processing the tobacco raw material, etc. Hereinafter, when the expression "increased vanillin content" or "high vanillin content" is used in this specification, it is intended to mean that the vanillin content is higher than that of a wild-type Nicotiana plant.

The tobacco raw material is a harvested material obtained from a Nicotiana plant into which a mutation inducing functional suppression of an endogenous cinnamyl alcohol dehydrogenase gene has been introduced. The vanillin content of the tobacco raw material is higher than the vanillin content of the same amount of tobacco raw material harvested from a wild-type Nicotiana plant.

The vanillin content of the harvested material used as the tobacco raw material may be at least twice the vanillin content of the same amount of harvested material obtained from a wild-type Nicotiana plant. Further, the vanillin content of the harvested material used as the tobacco raw material according to this embodiment may be at least 2.2 times, at least 6.4 times, or even higher than the vanillin content of the same amount of harvested material obtained from a wild-type Nicotiana plant.

The tobacco material according to this embodiment may comprise dried tobacco material as the tobacco raw material. The dried tobacco material is a dried product of a tobacco raw material harvested from a Nicotiana plant into which a mutation inducing functional suppression of an endogenous cinnamyl alcohol dehydrogenase gene has been introduced. The dried tobacco material of this embodiment has a higher vanillin content than dried tobacco material derived from a wild-type Nicotiana plant.

The dried tobacco material is obtained by drying the tobacco raw material, which is a harvested material of a Nicotiana plant. Any drying method may be used, including, for example, natural drying, warm-air drying, or hot-air drying, without limitation.

The vanillin content of the dried tobacco material may be at least 1.3 times the vanillin content of the same amount of dried tobacco material derived from a wild-type Nicotiana plant. Further, the dried tobacco material according to this embodiment may have a vanillin content that is at least 9 times, at least 25 times, or even higher than the vanillin content of the same amount of dried tobacco material derived from a wild-type Nicotiana plant.

The tobacco material may be obtained by processing the tobacco raw material or dried tobacco material by any method. For example, the tobacco raw material or dried tobacco material may be processed by means of drying, aging, extraction, blending, flavoring, high-temperature/high-pressure treatment, distillation, pulverization, or cutting, etc. The resulting tobacco material may be in the form of any of a cut filler, a powder, particles, a sheet, granules, or an extract. Such forms of tobacco material are preferred from the standpoint of applying the tobacco material to tobacco products.

The tobacco material may be in the form of a cut filler. Hereinafter, tobacco material in the form of cut filler is referred to as "raw material pieces". The particle size of the raw material pieces is preferably 0.5-1.18 mm. Such raw material pieces are obtained via sifting in accordance with JIS Z 8815 using a stainless steel sieve conforming to JIS Z 8801, for example. For example, 1) raw material pieces are sifted over a 20-minute period by means of dry mechanical shaking using a stainless steel sieve having 1.18 mm openings to obtain raw material pieces that pass through the stainless sieve having 1.18 mm openings. 2) The raw material pieces are then sifted over a 20-minute period by means of dry mechanical shaking using a stainless steel sieve having 0.50 mm openings to remove raw material pieces that pass through the stainless sieve having 0.50 mm openings. It is thus possible to prepare raw material pieces that pass through a stainless steel sieve of a specified upper limit (1.18 mm openings) and that do not pass through a stainless steel sieve of a specified lower limit (0.50 mm openings).

The tobacco material may be in the form of particles or powder. Hereinafter, tobacco material in the form of particles or powder is referred to as "tobacco particles". The tobacco particles comprise tobacco raw material or dried tobacco material chopped into even finer dimensions than the raw material pieces, and tobacco raw material or dried tobacco material which has been ground, etc. The particle size (D90) of the tobacco particles may be 10-1000 µm, for example, and is preferably 50-500 µm. Furthermore, the average particle size (D50) of the tobacco particles is preferably 20-1000 µm, and more preferably 50-500 µm. In this embodiment, the particle size (D90) and the average particle size (D50) of the tobacco particles are obtained by means of a laser diffraction scattering method. Specifically, the particle size (D90) and the average particle size (D50) of the tobacco particles are measured by using a laser diffraction particle size distribution measurement apparatus (e.g., LA-950 (tradename) available from HORIBA Ltd.). The amount of tobacco particles contained in 100 wt% of non-tobacco plant raw material-containing composition can be 0-90 wt%, and may be 1-80 wt%, 5-80 wt%, 10-70 wt%, or 20-70 wt%.

The tobacco material may be in the form of a sheet. Hereinafter, tobacco material in the form of a sheet is referred to as a "tobacco material-containing sheet". The tobacco material-containing sheet may be, for example, a sheet comprising a single tobacco material, a sheet comprising multiple tobacco materials, or a sheet comprising a tobacco material and a raw material from a plant other than a Nicotiana plant. Furthermore, these sheets may have a structure comprising a single sheet, multiple laminated sheets, or a crimped sheet, for example. Furthermore, the tobacco material-containing sheet may be formed from, for example, a layer containing the tobacco material, and a tobacco material-free layer which comprises a raw material derived from a plant other than a Nicotiana plant.

Any known tobacco, that is, plants other than those of the genus Nicotiana, may be used as plants other than a Nicotiana plant. For example, plants known to have characteristic aromas may be used from the standpoint of improving the smoking taste. Furthermore, as plant raw materials other than the Nicotiana plant, the whole plant may be used as-is, or the plant may be subjected to any processing, such as drying, pulverization, extraction, or granulation, etc.

The following methods may be used to produce the tobacco material-containing sheet. According to a first method, a paper sheet is produced by using a papermaking process. According to a second method, a suitable solvent such as water is combined with a mixture comprising dried tobacco material, a binder, and an aerosol source, etc. and homogenized, after which the homogenized material is thinly cast on a metal plate or a metal plate belt and dried, to produce a cast sheet. According to a third method, a suitable solvent such as water is mixed with dried tobacco material and homogenized, and the homogenized material is extruded into the form of a sheet and shaped to produce a calendered sheet. Details on the abovementioned types of homogenized sheets are disclosed in "Encyclopedia of Tobacco, Tobacco Academic Studies Center, March 31, 2009".

The tobacco material may be in the form of granules. Hereinafter, tobacco material in the form of granules is referred to as "tobacco material-containing granules". The tobacco material-containing granules may be substantially spherical granules. The average particle size (D50) of the tobacco material-containing granules can be 1000 µm or less, and may be 700 µm or less, 600 µm or less, or 550 µm or less, and may be 400 µm or more. The average particle size (D50) of the non-tobacco plant raw material-containing granules denotes the average particle size (D50) based on a volume-based particle size distribution measured by means of a laser diffraction/scattering particle size distribution measurement method. The measurement of the average particle size by means of a laser diffraction/scattering particle size distribution measurement method may be carried out in accordance with JIS Z8825:2013 (particle size analysis - laser diffraction/scattering method). The average particle size (D50) may be measured, for example, by using a laser diffraction particle size distribution measurement apparatus (e.g., LA-950 available from HORIBA Ltd.).

The tobacco material may be in the form of an extract. Hereinafter, tobacco material in the form of an extract is referred to as a "tobacco extract". A tobacco extract is a substance or mixture exhibiting flavor, which is extracted from tobacco. The tobacco extract can be prepared by known methods. The following methods may be cited by way of example. 1) A method in which tobacco raw material or dried tobacco material is extracted using an extraction medium to obtain a tobacco extract; 2) a method in which an extraction medium is added to tobacco raw material or dried tobacco material, the mixture is heated, and the vapor that is generated is collected; and 3) a method in which a heat-vaporized extraction medium is caused to pass through tobacco raw material or dried tobacco material, and the vapor that has passed through is collected. Extraction media that may be cited include water or a hydrophilic organic solvent such as an alcohol. In the method of 1), water is preferably used as the extraction medium in the interests of workability, etc. In the methods of 2) and 3), propylene glycol, glycerol, or an alcohol such as ethanol is preferably used as the extraction medium in the interests of work efficiency. Acids or alkalis can also be used, as required, for extraction. The liquid containing the tobacco extract and the extraction medium that is obtained by the extraction is called the tobacco extract liquid.

In one embodiment of the present invention, the dried tobacco material may be subjected to an alkali treatment (also referred to below as an "alkali heating treatment". Flavor components generated by way of the alkali treatment may be collected to prepare a tobacco extract liquid containing the tobacco extract and water. In this process, the flavor components are preferably extracted in the form of a gas from the alkali-treated dried tobacco material, and the gas is preferably introduced into water so that the flavor components migrate into the water. Alternatively, the solid tobacco material remaining after alkali treatment may itself be used to prepare dried tobacco material. In this case, the tobacco extract liquid is preferably mixed with the solid tobacco material to prepare the solid tobacco material. An example of alkali treatment includes adding an alkaline substance to tobacco particles so that the solid-liquid ratio (w/v) falls within the range of 1:5 to 1:20, heat-treating the mixture at 50-200 °C for 30-120 minutes under atmospheric pressure conditions, and then cooling the resulting reaction product.

The alkaline substance used in the alkali treatment is preferably an alkaline liquid such as a sodium hydroxide aqueous solution or a potassium carbonate aqueous solution, for example. In this process, the alkaline substance is supplied until the pH of the dried tobacco material is within a specific range. The pH is preferably 8.0 or greater, and more preferably 8.9-9.7. The pH of the dried tobacco material is the pH of the water when the dried tobacco material is mixed with 10 times the amount of water. Furthermore, the tobacco material subjected to the alkali treatment may further be subjected to a neutralization treatment by adding an acidic substance. Preferred acidic substances for use in the neutralization treatment include acidic liquids such as aqueous phosphoric acid solution and aqueous hydrochloric acid solution. In this process, the acidic substance is supplied until the pH of the dried tobacco material is within a specific range. The pH is preferably 6.0 or greater, and more preferably 6.5-8.0. The pH of the dried tobacco material is the pH of the water when the dried tobacco material is mixed with 10 times the amount of water.

In one embodiment of the present invention, the dried tobacco material may be subjected to a high-temperature/high-pressure treatment. As an example of a high-temperature/high-pressure treatment, a method such as disclosed in JP H10-66669 A may be used, for example. In such a method, a solvent is added to tobacco particles so that the solid-liquid ratio (w/v) falls within the range of 1:5 to 1:20, and the mixture is heated at 100-250 °C for 10-180 minutes while sealed under a pressure-controlled environment, then the resulting reaction product is returned to atmospheric pressure and cooled. The solvent may be water or a hydrophilic organic solvent such as an alcohol.

In one embodiment of the present invention, the dried tobacco material may be subjected to steam distillation. Examples of steam-distillation methods include general techniques such as those described in "Encyclopedia of Fragrances", p. 366 (Masao Fujimaki et al., Asakura Shoten, August 27, 1980). For example, after adding water to a distillation kettle and bringing the water to a boil, the dried tobacco material is placed into the kettle and distilled for several hours. The steam-distillate water obtained from the distillation is collected, an acid is added to the steam-distillate water to adjust the pH to 3.0-4.0, and salt is further added to reach a concentration of 20%. An extraction solvent is further added, liquid-liquid extraction is performed using a separating funnel, the solvent layer is collected, an aqueous NaOH solution (pH 9-10) is added to remove the acid, and the solvent layer is concentrated using an evaporator to obtain a dried solid. The water used for steam distillation may be subjected to filtration, deodorization, or decolorization, etc.

In one embodiment of the present invention, the dried tobacco material may be subjected to ethanol extraction. As an example of ethanol extraction, 20-99.5% ethanol may be added to the dried tobacco material so that the solid-liquid ratio (w/v) is 1:2 to 1:20, the dried tobacco material may be immersed in the ethanol at 4 °C-60 °C for 1 hour to several months, and an ethanol extract liquid may be obtained.

### [2. Tobacco product]

A tobacco product according to an embodiment of the present invention comprises the tobacco material described above. Specific examples of tobacco products that may be cited include cut tobacco products, cigar products, pipe-tobacco products, cigarettes (paper-rolled tobacco products), electronic-cigarette products, smokeless-tobacco products (including snus and snuff), and water-pipe tobacco products. In particular, the tobacco product may be any of an electronic-cigarette product, a cigarette, or a smokeless-tobacco product. Examples of electronic-cigarette products include, but are not limited to, non-combustion high-temperature heating-type tobacco products which employ, as an aerosol source, an aerosol generated by heating a tobacco portion containing a tobacco material; non-combustion low-temperature heating-type tobacco products which have an atomization unit, separate from the tobacco portion, for atomizing an aerosol source, and an aerosol which is generated by heating of the atomization unit passes through a tobacco portion, entraining a tobacco flavor to be inhaled; and non-heating tobacco products from which the flavor of a tobacco portion is inhaled, without heating of the tobacco portion, etc. In the present specification, non-combustion high-temperature heating tobacco products and non-combustion low-temperature heating tobacco products are collectively referred to as "aerosol-generating articles".

Preferred forms of the tobacco material for use in aerosol-generating articles or cigarettes include cut fillers, powders, particles, sheets, and granules. These forms of tobacco material may further be prepared and mixed by any method to form a tobacco material-containing composition. A flavor source for any flavor-generating article can be formed by using at least either the tobacco material itself or the tobacco material-containing composition. The shape of the flavor source is not particularly limited and may be, for example, granular, sheet-like, or block-like.

A "tobacco material-containing composition" comprises the tobacco material and may additionally comprise, for example, a binder, an aerosol source, fibers, and flavorings. Materials that may be contained in the tobacco raw material-containing composition are described below.

The tobacco material-containing composition comprises the tobacco material described above. The tobacco material contained in the tobacco material-containing composition may be in the form of tobacco particles or a tobacco extract. The amount of tobacco particles contained in 100 wt% of the tobacco material-containing composition may be 0-90 wt%, 1-80 wt%, 10-70 wt%, or 20-70 wt%. The amount of tobacco extract contained in 100 wt% of the tobacco material-containing composition may be 0-90 wt%, 10-80 wt%, 10-70 wt%, 15-50 wt%, or 20-40 wt%.

The tobacco material-containing composition may comprise any binder. Conventional binder components which are known in this technical field may be suitably used as the binder. Examples of binder components that may be used include cellulose derivatives, agar, guar gum, and pectin, etc. When the tobacco material-containing composition comprises a binder, the amount of binder contained in 100 wt% of tobacco plant raw material-containing composition may be 1-30 wt%, preferably 5-10 wt%, and more preferably 5-8 wt%.

The tobacco material-containing composition may comprise an aerosol source. Conventional aerosol sources which are known in this technical field may be suitably used as the aerosol source. When the tobacco material-containing composition comprises an aerosol source, the amount of aerosol source contained in 100 wt% of tobacco plant raw material-containing composition is preferably 1-50 wt%, and more preferably 10-30 wt%.

The tobacco material-containing composition may comprise fibers. Conventional fibers which are known in this technical field may be suitably used as the fibers. When the tobacco raw material-containing composition comprises fibers, the amount of fibers contained in 100 wt% of tobacco material-containing composition may be 2-15 wt%.

The tobacco material-containing composition may comprise a flavoring. Conventional aerosol sources which are known in this technical field may be suitably used as the flavoring. Moreover, a flavoring contained in a first flavor source and a flavoring contained in a second flavor source may be the same or different. When the tobacco material-containing composition comprises a flavoring, the amount of flavoring contained in 100 wt% of tobacco material-containing composition may be 5-20 wt%, and is preferably 9-13 wt%.

The flavor source used in the tobacco product according to an embodiment of the present invention may be the above-described forms of tobacco material and tobacco material-containing compositions themselves, or may be a mixture comprising a suitable combination thereof. For example, the flavor source may be a mixture obtained by mixing: a first flavor source comprising the abovementioned tobacco material in the form of tobacco particles or the abovementioned tobacco material-containing composition; and a second flavor source comprising the abovementioned tobacco material. Furthermore, for example, the flavor source according to this embodiment may comprise a first flavor source comprising a cut filler as the tobacco material, and a second flavor source comprising a tobacco material-containing sheet. Furthermore, the flavor source according to this embodiment may comprise a combination of tobacco materials in different forms, or tobacco material-containing compositions in different forms, for example, the flavor source may comprise, for the first flavor source, a cut filler or tobacco material-containing sheet as the tobacco material, and a second flavor source comprising tobacco particles may be adhered to the surface of the first flavor source. Here, the "first flavor source" and "second flavor source" are each present independently, and the flavor source is formed by physically mixing the two.

In the flavor source, a weight ratio of the first flavor source and the second flavor source may be 1:20-20:1, 1:10-10:1, or 1:5-5:1. In the flavor source, the weight of the first flavor source may be greater than the weight of the second flavor source. Alternatively, in the flavor source, the weight of the first flavor source may be smaller than the weight of the second flavor source.

The flavor source may comprise a sheet-form first flavor source and a sheet-form second flavor source. Furthermore, a sheet of the first flavor source and a sheet of the second flavor source may be laminated. Furthermore, a sheet of the first flavor source and a sheet of the second flavor source may be crimped in a stacked state. It should be noted that a sheet-form flavor source can be produced by using the abovementioned tobacco sheets.

The flavor source may comprise a particulate or granular first flavor source and a sheet-form second flavor source. Alternatively, the flavor source may comprise a sheet-form first flavor source and a particulate or granular second flavor source. In this case, either the sheet-form first flavor source or second flavor source may be folded, and the particulate or granular other of the first flavor source and the second flavor source may be interposed in gaps of the folded sheet-form first flavor source or second flavor source. It should be noted that a particulate or granular flavor source can be produced by using the above mentioned tobacco particles or tobacco material-containing granules.

The flavor source may comprise a particulate or granular first flavor source and a particulate or granular second flavor source. In this case, the average particle size of the particulate or granular first flavor source may be different from or substantially the same as the average particle size of the particulate or granular second flavor source. Furthermore, the average particle size of the first flavor source may be greater than the average particle size of the second flavor source. Alternatively, the average particle size of the first flavor source may be smaller than the average particle size of the second flavor source.

### [3. Nicotiana plant]

A mutation inducing functional suppression of an endogenous cinnamyl alcohol dehydrogenase gene is introduced into the Nicotiana plant according to an embodiment of the present invention. The vanillin content of a harvested material (leaf or stem) of this Nicotiana plant is higher than the vanillin content of a harvested material (leaf or stem) obtained from the same amount of a wild-type Nicotiana plant. By virtue of this feature, it is possible to realize a plant having a high vanillin content in leaf tobacco, dried leaves, and remaining stem, etc. thereof, by functional suppression of a single gene. For example, the vanillin content of a harvested material obtained from the Nicotiana plant according to this embodiment may be at least twice the vanillin content of the same amount of harvested material obtained from a wild-type Nicotiana plant. Further, the vanillin content of a harvested material obtained from the Nicotiana plant according to this embodiment may be at least 2.2 times, at least 6.4 times, or even higher than the vanillin content of the same amount of harvested material obtained from a wild-type Nicotiana plant.

The vanillin content of the harvested material of the Nicotiana plant may be 0.05 ppm or more, preferably 5 ppm or more, and more preferably 25 ppm or more. Furthermore, the vanillin content of the harvested material of the Nicotiana plant may be 5000 ppm or less, 2000 ppm or less, 1000 ppm or less, or 250 ppm or less. By virtue of such a Nicotiana plant, it is possible to further improve the smoking taste of a tobacco product employing same

There is no particular limitation as to the Nicotiana plant as long as it is a plant belonging to the genus Nicotiana, and examples thereof that may be cited include: Nicotiana acaulis, Nicotiana acuminata, Nicotiana acuminata var. multzjlora, Nicotiana africana, Nicotiana alata, Nicotiana amplexicaulis, Nicotiana arentsii, Nicotiana attenuata, Nicotiana benavidesii, Nicotiana benthamiana, Nicotiana bigelovii, Nicotiana bonariensis, Nicotiana cavicola, Nicotiana clevelandii, Nicotiana cordifolia, Nicotiana corymbosa, Nicotiana debneyi, Nicotiana excelsior, Nicotiana forgetiana, Nicotiana fragrans, Nicotiana glauca, Nicotiana glutinosa, Nicotiana goodspeedii, Nicotiana gossei, Nicotiana ingulba, Nicotiana kawakamii, Nicotiana knightiana, Nicotiana langsdorfi, Nicotiana linearis, Nicotiana longiflora, Nicotiana maritima, Nicotiana megalosiphon, Nicotiana miersii, Nicotiana noctiflora, Nicotiana nudicaulis, Nicotiana obtusifolia, Nicotiana occidentalis, Nicotiana occidentalis subsp. hesperis, Nicotiana otophora, Nicotiana paniculata, Nicotiana pauczjlora, Nicotiana petunioides, Nicotiana plumbaginifolia, Nicotiana quadrivalvis, Nicotiana raimondii, Nicotiana repanda, Nicotiana rosulata, Nicotiana rosulata subsp. ingulba, Nicotiana rotundifolia, Nicotiana rustica, Nicotiana setchellii, Nicotiana simulans, Nicotiana solanifolia, Nicotiana spegauinii, Nicotiana stocktonii, Nicotiana suaveolens, Nicotiana sylvestris, Nicotiana tabacum, Nicotiana thyrsiflora, Nicotiana tomentosa, Nicotiana tomentosiformis, Nicotiana trigonophylla, Nicotiana umbratica, Nicotiana undulata, Nicotiana velutina, Nicotiana wigandioides, and Nicotiana plant hybrids, etc. Among these, Nicotiana tabacum and Nicotiana rustica, which are used as raw materials in leaf tobacco production, are especially preferred. Nicotiana sylvestris can also be preferably used. The Nicotiana plant according to an embodiment of the present invention especially preferably belongs to Nicotiana tabacum or Nicotiana rustica.

An individual resulting from mutation or disruption of the above gene is referred to herein as a mutant of the Nicotiana plant (also simply described as a "mutant"). Among Nicotiana plants, Nicotiana tabacum is an amphidiploid and possesses both a genome derived from the parent plant Nicotiana sylvestris (also referred to as the "S genome") and a genome derived from the parent plant Nicotiana tomentosiformis (also referred to as the "T genome"). In Nicotiana tabacum, a gene represented by the same name is, in most cases, present in both the S genome and the T genome. In the case of Nicotiana tabacum, the above mutant may have the above-described mutation in either the S genome or the T genome. The mutant may also have the above-described mutation in both the S genome and the T genome. A mutation for loss of function may be a single mutation in one gene or may include multiple mutations, and the type of mutation is not an issue. In the case of Nicotiana tabacum, the mutant may have a mutation in any or all of the four alleles present as two alleles in each of the S genome and the T genome, and when mutations are present in multiple alleles, the mutations may be the same or different.

The functions of the CAD genes in both the S genome and the T genome may be specifically suppressed, but the function of the CAD gene in only either one of the S genome or the T genome may also be specifically suppressed. As used herein, "specific functional suppression of either one of the S genome and the T genome" refers to suppressing only the function of the CAD gene on one of the S genome and the T genome without suppressing the function of the CAD gene on the other genome. In order to specifically suppress the function of only the CAD gene present in either the S genome or the T genome, it is preferable to introduce a change in the nucleotide sequence only into the CAD gene of that one genome.

The Nicotiana plant according to one aspect of the present invention has functional suppression of an endogenous gene which comprises, as a coding region, a polynucleotide encoding a polypeptide comprising the amino acid sequence shown in SEQ ID NO: 1 or 2. SEQ ID NO: 1 shows the amino acid sequence of the endogenous cinnamyl alcohol dehydrogenase encoded by the S genome of Nicotiana tabacum (referred to herein as "NtCAD-S"), and SEQ ID NO: 2 shows the amino acid sequence of the endogenous cinnamyl alcohol dehydrogenase encoded by the T genome of Nicotiana tabacum (referred to herein as "NtCAD-T").

SEQ ID NO: 3 shows the CDS sequence of the NtCAD-S gene (encoding the amino acid shown in SEQ ID NO: 1). SEQ ID NO: 5 is the genomic DNA sequence of the NtCAD-S gene which comprises, as a coding region, the nucleotide sequence shown in SEQ ID NO: 3. SEQ ID NO: 4 shows the CDS sequence of the NtCAD-T gene (encoding the amino acid shown in SEQ ID NO: 2). SEQ ID NO: 6 is the genomic DNA sequence of the NtCAD-T gene which comprises, as a coding region, the nucleotide sequence shown in SEQ ID NO: 4.

The endogenous cinnamyl alcohol dehydrogenase gene in the Nicotiana plant according to one aspect of the present invention may be (a) an endogenous cinnamyl alcohol dehydrogenase gene comprising, as a coding region, a polynucleotide encoding a polypeptide having at least 90% sequence identity to the amino acid sequence shown in SEQ ID NO: 1. Furthermore, the endogenous cinnamyl alcohol dehydrogenase gene in the Nicotiana plant according to one aspect of the present invention may be (b) an endogenous cinnamyl alcohol dehydrogenase gene comprising, as a coding region, a polynucleotide encoding a polypeptide having at least 90% sequence identity to the amino acid sequence shown in SEQ ID NO: 2. This embodiment may satisfy one or all of the following conditions. A mutation inducing functional suppression of the endogenous gene (a) is present in the endogenous gene (a). A mutation inducing functional suppression of the endogenous gene (b) is not present in the endogenous gene (a). A mutation inducing functional suppression of the endogenous gene (b) is present in the endogenous gene (b). A mutation inducing functional suppression of the endogenous gene (a) is not present in the endogenous gene (b).

In a preferred embodiment, the Nicotiana plant has functional suppression of an endogenous gene which comprises, as a coding region, a polynucleotide encoding a polypeptide having 90% or more (91%, 92%, 93%, 94%, 95%, 96%, 97%, 98%, 99%, or 100%) sequence identity to the amino acid sequence shown in SEQ ID NO: 1 or 2 and having CAD activity. When two of the endogenous genes are present in the Nicotiana plant, the coding region in one of the endogenous genes encodes a polypeptide having the abovementioned sequence identity to the amino acid sequence shown in SEQ ID NO: 1 or 2.

As used in this specification, the term "sequence identity (of the amino acid sequence)" means the percentage of the relevant (amino acid) sequence matching a reference (amino acid) sequence. Here, the portions of the sequence that do not match are portions in which substitutions, additions, deletions, or insertions (of amino acid residues) are present.

Here, the description "a polypeptide having 90% or more sequence identity to the amino acid sequence shown in ..." used to specify a polypeptide using the amino acid sequence listed in the Sequence Listing may also refer to a polypeptide normally present in a Nicotiana plant. In this specification, "polypeptide" and "protein" have substantially the same meaning and may be used interchangeably.

Accordingly, the specific polypeptide which is present in a smaller amount in the Nicotiana plant of the present invention should be a polypeptide having 90% or more sequence identity to each of the amino acid sequences shown in the Sequence Listing, and the sequence identity is preferably a higher percentage (e.g., 97% or more).

The Nicotiana plant according to one aspect of the present invention has functional suppression of an endogenous gene comprising, as a coding region, a polynucleotide encoding a polypeptide composed of an amino acid sequence in which one or more amino acids in the amino acid sequence shown in SEQ ID NO: 1 or 2 are deleted, substituted, or added, and having CAD activity. Here, the number of amino acids deleted, substituted, or added in each amino acid sequence is, for example, 1-30, 1-25, 1-20, 1-15, 1-10, 1-9, 1-8, 1-7, 1-6, 1-5, 1-4, 1-3, 1-2, or 1.

The Nicotiana plant according to one aspect of the present invention has functional suppression of an endogenous gene comprising, as a coding region, a polynucleotide composed of the nucleotide sequence shown in SEQ ID NO: 3 or 4.

The Nicotiana plant according to one aspect of the present invention has functional suppression of an endogenous gene comprising, as a coding region, a polynucleotide which hybridizes under stringent conditions with a polynucleotide composed of a nucleotide sequence complementary to a polynucleotide composed of the nucleotide sequence shown in SEQ ID NO: 3 or 4, and which encodes a polypeptide having CAD activity.

The Nicotiana plant according to one aspect of the present invention has functional suppression of an endogenous gene which comprises, as a coding region, a polynucleotide having 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more sequence identity to the nucleotide sequence shown in SEQ ID NO: 3 or 4, and which encodes a polypeptide having CAD activity.

A polynucleotide encoding the amino acids shown in SEQ ID NO: 1 also encompasses degenerate sequences of the nucleotide sequence shown in SEQ ID NO: 3 due to the degeneracy of the genetic code. A polynucleotide encoding the amino acids shown in SEQ ID NO: 2 also encompasses degenerate sequences of the nucleotide sequence shown in SEQ ID NO: 4 due to the degeneracy of the genetic code.

### (Mutation)

As used in this specification, "mutation" has the meaning ordinarily understood in the technical field to which the present application belongs, and means, for example, any change (for example, substitution, deletion, insertion, addition, duplication, inversion, or translocation) in a nucleotide present on the wild-type genome or in an amino acid residue present in a wild-type polypeptide. Accordingly, "mutation of an endogenous gene" means: a mutation of the gene, in which the original functional polypeptide is not produced (including mutations that produce a polypeptide with reduced or lost function); a mutation of the gene, in which the polypeptide is produced but a smaller amount is produced; a mutation of the gene, in which the polypeptide is produced but stability of the polypeptide is reduced; loss of the gene (a genomic DNA sequence including the coding region or untranslated regions); or a mutation in which transcription from the gene is suppressed (such as deletion of a transcriptional regulatory region or transcription start region).

A mutation may be present in at least one of a promoter sequence (a sequence located upstream (5' side) relative to the coding region), a terminator sequence (a sequence located downstream (3' side) relative to the coding region), the 5' untranslated region and 3' untranslated region, conserved sequences at both ends of an intron (for example, GT at the 5' end and AG at the 3' end), or a coding region. In particular, the mutation may be present within the coding region of the endogenous cinnamyl alcohol dehydrogenase gene. When a mutation is present in at least one of a promoter sequence, terminator sequence, or coding region, the mutation may be any one of substitution, deletion, insertion, addition, duplication, inversion, or translocation. In particular, there may also be functional suppression of an endogenous gene comprising, as a coding region, a polynucleotide encoding a polypeptide composed of an amino acid sequence in which one or more amino acids in the amino acid sequence of the CAD polypeptide are deleted, substituted, or added, and having CAD activity. Here, the number of amino acids deleted, substituted, or added in each amino acid sequence is, for example, 1-30, 1-25, 1-20, 1-15, 1-10, 1-9, 1-8, 1-7, 1-6, 1-5, 1-4, 1-3, 1-2, or 1.

The Nicotiana plant may also have a mutation introduced outside the coding region of the endogenous cinnamyl alcohol dehydrogenase gene, which induces functional suppression of the endogenous cinnamyl alcohol dehydrogenase gene. When the mutation is introduced outside the coding region of the endogenous cinnamyl alcohol dehydrogenase gene, the mutation may be insertion of a polynucleotide that expresses an antisense RNA molecule, an RNAi molecule, or a co-suppression molecule that promotes degradation of the mRNA transcribed from the endogenous cinnamyl alcohol dehydrogenase gene.

When function is lost due to substitution, the substitution may be present in a promoter sequence, terminator sequence, 5' untranslated region, 3' untranslated region, conserved sequences at both ends of an intron, or at least one coding region.

For example, substitution in a nucleotide sequence important for regulation of gene expression, located in the promoter sequence or in the 5' untranslated region or 3' untranslated region of a gene, results in decreased transcriptional activity of the gene or decreased stability of the transcript from the gene. Either of these decreases can result in decreased translation products due to the accompanying decrease in transcripts from the gene. Substitution in the conserved sequences of an intron (a splice mutation) produces abnormal mRNA in which an unnecessary intron is added or inserted, by causing abnormal splicing of the mRNA. Abnormal mRNA produces an abnormal translation product, for example due to a frameshift, or fails to terminate translation.

When a nucleotide substitution in the coding region is a missense mutation (which causes a decrease in the amount of wild-type polypeptide present), the substitution results in an amino acid different from the original amino acid, and can therefore produce a polypeptide whereof the original function is reduced or lost.

A substitution in the coding region can also produce an incomplete translation product or a translation product that does not retain its original function. An incomplete translation product arises from conversion of a codon that encodes an amino acid into a stop codon by the substitution (a nonsense mutation). An incomplete translation product lacks one or more consecutive amino acid residues including amino acid residues at the C-terminus, as compared with the original translation product. A nonsense mutation occurs in any codon located upstream of the original stop codon, and is located upstream with at least one codon between it and the original stop codon. Accordingly, a translation product from a gene having a nonsense mutation is incomplete in length. A translation product that has lost its original function is produced by an amino acid substitution. In this case, the amount of transcript may be the same as in a wild-type plant. The translation product undergoes a change in its three-dimensional structure, or reduction of the function of a functional domain, or the like. One preferred aspect of the mutation of the present invention is an amino acid substitution that produces such a translation product with impaired original function. The amino acid substitution is preferably a non-conservative substitution, which has a high likelihood of altering the function of the translation product. Examples of non-conservative substitutions that may be cited include substitutions to amino acids with different charge or hydrophobicity (for example, substitution from a basic amino acid to an acidic amino acid, from a basic or acidic amino acid to a neutral amino acid, from a neutral amino acid to a basic or acidic amino acid, or from a polar amino acid to a non-polar amino acid), and substitutions to amino acids having side chains of different bulk (steric size).

As another example of phenomena caused by a nonsense mutation, when a nonsense mutation is present in the protein-coding region of the CAD gene, nonsense-mediated mRNA decay (Brogna and Wen (2009) Nat. Structural Mol. Biol. 16:107-113) can occur. Nonsense-mediated mRNA decay causes degradation of the transcript, and therefore a nonsense mutation may result in a decrease in transcript levels.

For nonsense-mediated mRNA decay to occur, at least one exon having a nonsense mutation is preferably present in the CAD gene. More preferably, the exon having the nonsense mutation is not the most downstream (3'-side) exon that constitutes the CAD gene. The CAD gene of wild-type Nicotiana plants comprises five exons and four introns. Accordingly, a preferred embodiment of a nonsense mutation that causes nonsense-mediated mRNA decay is that at least one nonsense mutation is present in exon 1 to exon 4 of the CAD gene.

Mutations other than substitutions (such as deletions or insertions) occurring in the promoter sequence, the 5' untranslated region, and/or the 3' untranslated region can, like substitutions, result in reduced transcript levels due to decreased transcriptional activity or stability, and reduced amounts of the polypeptide. Mutations other than substitutions in the conserved sequences of introns can also, like substitutions, produce translation of a polypeptide having an amino acid sequence different from the original. Mutations other than substitutions in the coding region can also produce translation of a polypeptide having an amino acid sequence different from the original, due to deletion or insertion of amino acid residues (arising from deletion or insertion of consecutive nucleotides in multiples of three), or due to a frameshift. In addition, a large deletion including the entire gene or insertion of a large fragment into the gene can produce loss of expression of the gene itself.

The above mutations may be introduced by mutagen treatment, genome editing, or gene knockout. In particular, the mutation may be introduced by the mutagen treatment described above.

The mutagen treatment of the gene can be carried out by artificially applying the above mutagen to the Nicotiana plant (and, if necessary, in combination with suppression of DNA repair functions). Examples of mutagens that may be used include chemical agents such as ethyl methanesulfonate (EMS), sodium azide, ethidium bromide, and nitrous acid, but the mutagen is not limited to these as long as it is a chemical agent that induces mutations in the genomic DNA of a Nicotiana plant. Examples of mutagens also include radiation such as γ-rays, heavy-ion beams, X-rays, neutron rays, or UV, but the mutagen is not limited to these as long as it is radiation etc. that induces mutations in the genomic DNA of a Nicotiana plant. A preferred mutagen is EMS. These methods are preferred from the standpoint that no exogenous factor needs to be added to the target plant. Recombination of the above gene can be carried out by homologously recombining part or all of the target gene with a recombinant sequence according to known gene recombination methods. Genome editing of the gene can be carried out by known techniques (for example, zinc-finger nucleases (ZFN), transcription activator-like effector nucleases (TALEN), and the CRISPR/Cas9 system). Knockout of the above gene can be carried out by insertion of a known transposon (mobile genetic element) or T-DNA, etc.

A nucleotide substitution in the CAD gene due to EMS treatment produces, for example, (I) a frameshift mutation, (II) a truncation mutation (in which N-terminal amino acid residues are necessarily deleted), (III) a splice mutation, or (IV) a nonsense mutation. This is because EMS treatment tends to cause specific nucleotide changes (C→T substitutions and G→A substitutions) in DNA. Examples of substitutions that may occur in the CAD gene due to EMS treatment include the following: when the third G of the ATG nucleotide sequence corresponding to the translation-initiation codon methionine (Met, also written as M) is substituted with A, (I) a frameshift mutation or (II) a truncation mutation can occur. Also, when the second G and/or third G of the TGG nucleotide sequence corresponding to tryptophan (Trp, also written as W) is substituted with A, or when the first C of the CAA or CAG nucleotide sequence corresponding to glutamine (Gln, also written as Q), or of CGA corresponding to arginine (Arg, also written as R), is substituted with T, (IV) a nonsense mutation can occur. When the G of the GT sequence or of the AG sequence located at the intron boundary sequences is substituted with A, (III) a splice mutation can occur.

In the CRISPR/Cas9 system, genome editing is possible when the guide RNA and Cas9 protein are present in the target cell; in TALEN and ZFN, genome editing is possible when the fusion protein (in which a DNA-binding domain and a nuclease are fused) is present in the target cell. Accordingly, the above guide RNA and Cas9 protein, and the above fusion proteins, can all be directly introduced into the target cell. Examples of methods for directly introducing these into the target cell that may be cited include the PEG method, electroporation, and particle bombardment. A vector having an inserted construct (a polynucleotide encoding the guide RNA and Cas9 protein, and optionally including a promoter and/or terminator) may also be introduced into the target cell or tissue via Agrobacterium or the like.

In the CRISPR/Cas9 system, a sequence complementary to the nucleotide sequence located immediately upstream of XGG on the genome forms base pairs with part of the guide RNA, and within that nucleotide sequence the double-stranded genomic DNA is cleaved by Cas9.

In TALEN, each of the pair of DNA-binding domains of the artificial nuclease that forms a dimer binds to a nucleotide sequence located at both ends of the FokI cleavage domain, with a spacer of 5-20 nucleotides therebetween. The nucleotide sequences are present on one strand and the other strand of the double-stranded genomic DNA, and accordingly, one of the pair of DNA-binding domains binds to one strand, and the other binds to the other strand. The DNA-binding domains are composed of modules (repeat units) of 33-34 amino acid residues, and the number of modules corresponds to the number of nucleotides to be bound.

In ZFN, like TALEN, each of the pair of DNA-binding domains of the artificial nuclease that forms a dimer binds to a nucleotide sequence located at both ends of the FokI cleavage domain, with a spacer of 5-20 nucleotides therebetween. The DNA-binding domains are composed of multiple zinc-finger modules.

Nicotiana plants in which mutations have been artificially induced have thus been described as above, but the invention is not limited to these. For example, in a Nicotiana plant, mutation or disruption of a gene may also arise from spontaneous mutation. Spontaneous mutations of genes generally arise from replication errors and gene damage. Causes of such damage include exposure to naturally occurring known mutagens (for example, radiation or ultraviolet light, etc.).

The various mutations described above can be easily introduced into a Nicotiana plant by a person skilled in the art. That is, based on these sequence data, the region in the genome of various Nicotiana plants encompassed within the concept of the present invention into which a mutation should be introduced can be appropriately determined.

Mutation or disruption of a gene can be determined by detecting the presence or absence of a mutation in the gene. Examples of methods for detecting a mutation in a gene include: (1) a method of amplifying the DNA sequence containing the mutation by PCR or the like, and then directly determining the DNA nucleotide sequence using a commercially available sequencer; (2) a method of detecting sequence differences by differences in electrophoresis distance using the SSCP (Single Strand Conformation Polymorphism) method; (3) a method of detecting SNPs (Single Nucleotide Polymorphisms) using the Cycleave PCR method; (4) a method of detecting the presence or absence of a mutation by cleaving mismatch sites using T7 Endonuclease I or the like; (5) the CAPS (Cleaved Amplified Polymorphic Sequence) method, in which the presence or absence of cleavage by restriction enzyme treatment allows discrimination of the presence or absence of a mutation; (6) the dCAPS (derived CAPS) method, in which a primer set intentionally containing a mismatch is used so that the presence or absence of cleavage by a restriction enzyme allows discrimination of the presence or absence of a mutation; (7) a method of detecting the presence or absence of a mutation by detecting whether a probe that specifically hybridizes to the mutant sequence has hybridized (PCR using a TaqMan probe); (8) a method of performing single-base extension using a primer adjacent to the mutation, and detecting the presence or absence of the mutation by the mass difference of the incorporated nucleotide (MassARRAY analysis); and (9) in the case of deletions or insertions, a method of detecting the mutation by differences in electrophoretic mobility; however, any method may be used as long as it allows discrimination of the presence or absence of a mutation. Alternatively, mutation or disruption of a gene can be determined by comparing the size or expression level of the protein produced as a result of the gene modification with those of the wild-type protein. Specifically, such comparison can be performed, for example, by Western blotting.

Suppression of expression of the above gene includes suppression of transcription from the gene to mRNA, suppression of translation from the gene to the polypeptide through the mRNA (for example, degradation of the mRNA), and suppression of the function of the translated polypeptide. Degradation of mRNA can arise from the abovementioned nonsense-mediated mRNA decay. Suppression of transcription can be achieved by inhibition of transcription factors that promote transcription from the gene, or by inhibition of access of transcription initiation factors to the gene. Suppression of translation can be achieved using antisense RNA molecules, RNAi molecules, or co-suppression molecules. Suppression of the function of the polypeptide can be achieved by molecules that bind to the functional polypeptide and thereby inhibit functions of the polypeptide (for example, decoy nucleic acids, ribozymes, antibodies, and inhibitory peptides).

As vectors used for transformation of Nicotiana plants for the purpose of suppressing gene expression or introducing mutations into a gene, any vector may be used as long as it is capable of expressing the polynucleotide inserted into the vector within plant cells. Examples of such vectors include pBI-type, pPZP-type, and pSMA-type vectors, which can introduce the target polynucleotide into plant cells via Agrobacterium. In particular, plasmids of binary vector systems (such as pBIG, pBIN19, pBI101, pBI121, and pPZP202) are preferred.

When suppression of gene expression is achieved by RNAi, the trigger sequence used to suppress expression of the target gene by RNAi is inserted into the above vector as a mutation. The trigger sequence is, for example, a polynucleotide represented by a nucleotide sequence of at least 21-30 consecutive nucleotides, which is part of a polynucleotide (that may have 0.1-1% substitutions) encoding a polypeptide having the amino acid sequence shown in SEQ ID NO: 1 or 2) (for example, 21 or more, 22 or more, 23 or more, 24 or more, 25 or more, 26 or more, 27 or more, 28 or more, 29 or more, or 30 or more consecutive nucleotides) (sense RNA portion), and a polynucleotide represented by a nucleotide sequence complementary to that polynucleotide (antisense RNA portion). The nucleotide sequence of "at least 21-30 consecutive nucleotides" more specifically means a nucleotide sequence of 21 or more, 23 or more, 25 or more, 30 or more, 35 or more, 40 or more, 45 or more, 50 or more, 60 or more, 70 or more, 80 or more, 90 or more, or 100 or more consecutive nucleotides.

The abovementioned suppression (of transcription, translation, or polypeptide function) can be achieved, for example, by directly introducing into the plant a molecule for achieving the suppression, or by introducing into the plant a nucleic acid molecule that encodes the molecule (transformation of the plant). As a result of transformation of the plant, the nucleic acid molecule is incorporated into one or more arbitrary regions of the genome of the plant. In the case of Nicotiana tabacum, which is an amphidiploid, the suppression does not require that the nucleic acid molecule be incorporated into both the S genome and the T genome, as long as the suppression is achieved.

### (Functional suppression)

The Nicotiana plant according to one aspect of the present invention has functional suppression of an endogenous gene comprising, as a coding region, a polynucleotide composed of the nucleotide sequence of the CAD gene. The term "functional suppression of an endogenous gene" as used in this specification means a state in which a gene present on the genome does not exhibit its original function. Accordingly, "functional suppression of an endogenous gene" is a term that encompasses "mutation of the endogenous gene", "disruption of the endogenous gene", and "suppression of expression of the endogenous gene" by something other than the endogenous gene itself (including exogenous genes). Further, "specifically inducing functional suppression" means suppressing only the function of the target gene without suppressing the function of any gene other than the target gene. For example, it is desirable to avoid a situation in which functional suppression of the cinnamyl alcohol dehydrogenase gene simultaneously suppresses the functions of multiple genes under the control of the same transcription factor, thereby causing metabolic abnormalities.

In the Nicotiana plant according to one embodiment of the present invention, the functional suppression may be a decrease, relative to a wild-type plant, in the amount of the original functional polypeptide translated from the coding region of the endogenous cinnamyl alcohol dehydrogenase gene.

"Decrease in the amount" of the polypeptide means that, relative to the amount of wild-type polypeptide, the amount of the polypeptide is 70% or less, 60% or less, 50% or less, 40% or less, 30% or less, 20% or less, 10% or less, 5% or less, or 1% or less. The amount of the polypeptide, relative to the amount of the wild-type polypeptide, may be appropriately selected from the above values so as to result in an increase in the vanillin content of the Nicotiana plant.

The decrease in the amount of the polypeptide in the Nicotiana plant according to the present invention is preferably genetically stably inherited in cultured cells, callus, protoplasts, seeds, and progeny obtained from the Nicotiana plant. Accordingly, the Nicotiana plant according to the present invention may be an individual that has developed from cultured cells, callus, protoplasts, seeds, or progeny generated through artificial manipulation, and these materials for obtaining such an individual fall within the scope of the present invention.

The functional suppression may also be a decrease, relative to a wild-type plant, in translation of the original functional polypeptide. Translation of the polypeptide occurs on the basis of a decrease in mRNA (caused by factors such as instability of the mRNA itself, promotion of mRNA degradation, suppression of transcription of the mRNA, or other factors affecting the amount of mRNA) or a decrease in the amount of translation from the mRNA (due to factors such as deficiency of translation components (tRNA and ribosomes), inhibition of recruitment, or functional defects).

The functional suppression may also be a decrease, relative to a wild-type plant, in the amount of mRNA transcribed from the endogenous cinnamyl alcohol dehydrogenase gene. A decrease in the amount of mRNA transcription arises, for example, from suppression of transcription from the endogenous gene to mRNA. Suppression of transcription can be achieved by inhibition of access of transcription initiation factors to the endogenous gene, which arises as a result of introducing a mutation into the endogenous gene.

The functional suppression may also be promotion of degradation of the mRNA transcribed from the endogenous gene. Degradation of mRNA can be caused by generation of abnormal mRNA (which undergoes nonsense-mediated mRNA decay), the presence of exogenous factors that degrade mRNA, activation of endogenous components that degrade mRNA, or the presence of degradation-promoting sequences in the mRNA. In a Nicotiana plant, when degradation of the mRNA transcribed from the endogenous gene is promoted, there is a decrease in mRNA in the plant. That is, in the Nicotiana plant, the functional suppression may be a decrease, relative to a wild-type plant, in the amount of mRNA transcribed from the endogenous gene. Here, "decrease in the amount of mRNA transcribed from the endogenous gene" means that, relative to the amount of the transcript of the endogenous gene in a wild-type plant, the amount of the transcript is 70% or less, 60% or less, 50% or less, 40% or less, 30% or less, 20% or less, 10% or less, 5% or less, or 1% or less.

In the Nicotiana plant, the mutation may also be insertion, outside the region in which the endogenous gene is present, of a polynucleotide that expresses a factor promoting degradation of the mRNA transcribed from the endogenous gene. The factor may be an antisense RNA molecule, an RNAi molecule, or a co-suppression molecule.

The Nicotiana plant according to one aspect of the present invention has functional suppression of an endogenous gene comprising, as a coding region, a polynucleotide which hybridizes under stringent conditions with a polynucleotide composed of a nucleotide sequence complementary to a polynucleotide composed of the nucleotide sequence encoding the endogenous CAD gene, and which encodes a polypeptide having CAD activity.

Stringent conditions refer to conditions under which double-stranded polynucleotides specific to a given nucleotide sequence are formed, but formation of non-specific double-stranded polynucleotides is markedly suppressed. In other words, stringent conditions may be described as conditions under which hybridization occurs at a temperature within a range up to 15°C, preferably 10°C, more preferably 5°C below the melting temperature (Tm value) of a hybrid between nucleic acids having high homology, e.g., a double-stranded polynucleotide that is completely matched to the probe. For example, hybridization conditions that may be cited include hybridizing for 20 hours at 68°C in a general hybridization buffer. As one example, hybridization may be carried out for 16-24 hours at a temperature of 60-68°C, preferably 65°C, more preferably 68°C, in a buffer containing 0.25 M Na2HPO4, pH 7.2, 7% SDS, 1 mM EDTA, and 1× Denhardt's solution, followed by two 15-minute washes at 60-68°C, preferably 65°C, more preferably 68°C, in a buffer containing 20 mM Na2HPO4, pH 7.2, 1% SDS, and 1 mM EDTA. As another example, pre-hybridization is performed overnight at 42°C in a hybridization solution containing 25% formamide (or 50% formamide under more stringent conditions), 4× SSC (sodium chloride/sodium citrate), 50 mM Hepes pH 7.0, 10× Denhardt's solution, and 20 µg/mL denatured salmon sperm DNA, after which hybridization is performed by adding a labeled probe and incubating overnight at 42°C. The subsequent washing may be carried out with washing liquid and temperature conditions of the order of "1× SSC, 0.1% SDS, 37°C", more stringent conditions of the order of "0.5× SSC, 0.1% SDS, 42°C", and even more stringent conditions of the order of "0.2× SSC, 0.1% SDS, 65°C". As the washing conditions in the hybridization become more stringent, isolation of DNA having higher homology to the probe sequence can be expected. However, the combinations of SSC, SDS, and temperature conditions described above are merely examples, and a person skilled in the art will be able to achieve similar stringency by appropriately combining these or other factors (for example, probe concentration, probe length, hybridization reaction time, etc.) which determine the stringency of hybridization. For example, a person skilled in the art can readily obtain such genes by referring to Molecular Cloning (Sambrook, J. et al., Molecular Cloning: A Laboratory Manual 2nd ed., Cold Spring Harbor Laboratory Press, 10 Skyline Drive Plainview, NY (1989)).

### [4. Processing of tobacco raw material]

In one embodiment of the present invention, the tobacco raw material may be alkali-treated. Flavor components generated by way of the alkali treatment may be collected to prepare a tobacco extract liquid containing the tobacco extract and water. In this process, the flavor components are preferably extracted in the form of a gas from the alkali-treated tobacco raw material, and the gas is preferably introduced into water so that the flavor components migrate into the water.

The alkaline substance used in the alkali treatment is preferably an alkaline liquid such as a sodium hydroxide aqueous solution or a potassium carbonate aqueous solution, for example. In this process, the alkaline substance is supplied until the pH of the tobacco raw material is within a specific range. The pH is preferably 8.0 or greater, and more preferably 8.9-9.7. The pH of the tobacco raw material is the pH of the water when the tobacco raw material is mixed with 10 times the amount of water.

In one embodiment of the present invention, the tobacco raw material may be subjected to a high-temperature/high-pressure treatment. As an example of a high-temperature/high-pressure treatment, a method such as disclosed in JP H10-66669 A may be used, for example. In such a method, a solvent is added to tobacco particles so that the solid-liquid ratio (w/v) falls within the range of 1:5 to 1:20, and the mixture is heated at 100-250 °C for 10-180 minutes while sealed under a pressure-controlled environment, then the resulting reaction product is returned to atmospheric pressure and cooled. The solvent may be water or a hydrophilic organic solvent such as an alcohol.

In one embodiment of the present invention, the tobacco raw material may be subjected to steam distillation. Examples of steam-distillation methods include general techniques such as those described in "Encyclopedia of Fragrances", p. 366 (Masao Fujimaki et al., Asakura Shoten, August 27, 1980). For example, after adding water to a distillation kettle and bringing the water to a boil, the tobacco raw material is placed into the kettle and distilled for several hours. The steam-distillate water obtained from the distillation is collected, an acid is added to the steam-distillate water to adjust the pH to 3.0-4.0, and salt is further added to reach a concentration of 20%. An extraction solvent is further added, liquid-liquid extraction is performed using a separating funnel, the solvent layer is collected, an aqueous NaOH solution (pH 9-10) is added to remove the acid, and the solvent layer is concentrated using an evaporator to obtain a dried solid. The water used for steam distillation may be subjected to filtration, deodorization, or decolorization, etc.

In one embodiment of the present invention, the tobacco raw material may be subjected to ethanol extraction. As an example of ethanol extraction, 20-99.5% ethanol may be added to the tobacco raw material so that the solid-liquid ratio (w/v) is 1:2 to 1:20, the dried tobacco material may be immersed in the ethanol at 4 °C-60 °C for 1 hour to several months, and an ethanol extract liquid may be obtained.

### [5. Method for producing Nicotiana plant]

One embodiment of the present invention comprises a step of introducing a mutation specifically inducing functional suppression of an endogenous cinnamyl alcohol dehydrogenase gene into this endogenous gene in the genome of the Nicotiana plant. Details of the mutation introduced into the Nicotiana plant are described in detail in the section [3. Nicotiana plant].

The introduction step may comprise a step of introducing the mutation into the coding region of the endogenous cinnamyl alcohol dehydrogenase gene. The introduction step may also be carried out by mutagen treatment, genome editing, or gene knockout. The introduction step may especially be mutagen treatment.

The introduction step may also comprise a step of introducing, into the genome of the Nicotiana plant, a mutation specifically inducing functional suppression of the endogenous cinnamyl alcohol dehydrogenase gene, outside the coding region of the endogenous cinnamyl alcohol dehydrogenase gene. The functional suppression is a decrease, relative to a wild-type plant, in the amount of mRNA transcribed from the endogenous cinnamyl alcohol dehydrogenase gene. The functional suppression is more preferably promotion of degradation of the mRNA transcribed from the endogenous gene. The introduction step may further comprise insertion of a polynucleotide that expresses an antisense RNA molecule, an RNAi molecule, or a co-suppression molecule that promotes degradation of the mRNA transcribed from the endogenous cinnamyl alcohol dehydrogenase gene.

In the production method, an individual exhibiting a desired phenotype may further be selected from a mutant population of plants having mutations. As an example of selecting individuals, a procedure is described for selecting desired individuals from a mutant population (panel) obtained when treatment with a mutagen is used.

As one example of a method for producing a Nicotiana plant having a mutation in the CAD gene, there may be cited a method in which, as described above, a Nicotiana plant is treated with a mutagen such as EMS, a mutant population (panel) of Nicotiana plants in which mutations have been induced throughout the genome of the Nicotiana plants is produced, and genomic DNA is extracted. Using a primer specific to the gene, the CAD gene is amplified from each genomic DNA sample of the panel, or from pooled samples thereof, the nucleotide sequence of the amplification product is determined, and lines containing homozygous mutations are selected. In the case of a Nicotiana plant, lines containing homozygous mutations in the S-genome and lines containing homozygous mutations in the T-genome are each acquired, and an F1 is produced by crossing same. Further, the selfed progeny (F2) are grown, and from among them, lines containing homozygous mutations in both the S-genome and the T-genome are acquired (because of two-factor recessive inheritance, they are obtained with a probability of 1/16).

Selection of individuals exhibiting the desired phenotype may be carried out based on vanillin content. For example, it is possible to select individuals in which the vanillin content of the tobacco material or tobacco raw material contained in the product is increased in comparison with the vanillin content of wild-type tobacco material or tobacco raw material.

Accordingly, the method according to the embodiment above may further comprise one or more of the following steps: • a step of producing a Nicotiana plant population (panel) in which mutations have been induced throughout the Nicotiana plant genome;
- a step of extracting genomic DNA from lines included in the panel;
- a step of determining the nucleotide sequence of the CAD gene in the genomic DNA;
- a step of selecting, from the panel, lines containing homozygous mutations; and
- a step of confirming metabolites or CAD activity in the lines, or in tobacco material or tobacco raw material containing these lines.

The lines may be crossed with lines that have not been subjected to mutagen treatment at any time prior to carrying out the step of confirming measurement of metabolites or CAD activity. Crossing makes it possible to eliminate mutations that may exist outside the CAD gene. In certain embodiments, the lines having mutations in the CAD gene may be backcrossed multiple times with lines that have not been subjected to mutagen treatment (the original lines used to produce the panel).

Extraction of genomic DNA from Nicotiana plant mutants may be carried out according to known methods, and commercially available extraction kits may also be used. The genomic DNA may be a crude preparation, or it may be a purified product obtained through several purification steps.

Amplification of polynucleotides can be performed, for example, by PCR, but may also be carried out by other known gene amplification methods, such as LCR (ligase chain reaction) or LAMP (Loop-Mediated Isothermal Amplification).

Primer sequences for amplifying each polynucleotide can be designed, for example, from the nucleotide sequence. For example, in the case of a Nicotiana plant, based on the results of homology analysis between the nucleotide sequence of SEQ ID NO: 5 (genomic sequence of the S-type CAD gene) and the nucleotide sequence of SEQ ID NO: 6 (genomic sequence of the T-type CAD gene), S-type-specific regions and T-type-specific regions are first identified. By designing primers in those regions, the S-type and T-type CAD genes can each be specifically amplified from the extracted genomic DNA (containing S-type and T-type). The design site may be selected from S-type-specific or T-type-specific regions, but is preferably an intron, a 5' untranslated region, or a 3' untranslated region. The primer length is preferably 15-30 nucleotides, and more preferably 17-25 nucleotides. The primer sequence may be designed on the basis of the sequence in a region specific to one nucleotide sequence or in a region common to both nucleotide sequences. Furthermore, as long as the primer can function as a primer for amplifying a sequence of a predetermined number of nucleotides containing the mutation site, the sequence may contain one or more substitutions, deletions, and/or additions. The primer may also be labeled with a fluorescent substance or radioactive substance, etc. as required.

The length of each polynucleotide to be amplified is not particularly limited as long as it is a length suitable for the various detection methods to be described later, but is, for example, 20-5000 nucleotides, more preferably 50-2000 nucleotides, even more preferably 100-700 nucleotides, and still more preferably 100-500 nucleotides.

A mutant Nicotiana plant having functional loss and having mutations in two alleles (in the case of Nicotiana tabacum, a total of four alleles including both the T-genome and S-genome alleles) can be obtained by the following method, for example. As described above, the Nicotiana plant is treated with a mutagen, a mutant population (panel) in which mutations have been induced throughout the genome is produced, and genomic DNA is extracted. Using gene-specific primers, the target gene (polynucleotide) is amplified from the genomic DNA of the panel, the nucleotide sequence of the amplification product is determined, and lines having homozygous mutations are selected. For example, in the case of Nicotiana tabacum, lines (M2) having homozygous mutations in each of the S-genome and T-genome are first of all obtained, and an F1 is produced by crossing same. Further, the selfed progeny (F2) are grown, and from among them, lines having homozygous mutations in both the S-genome and the T-genome are obtained. In order to obtain a mutant Nicotiana plant having functional loss and having a mutation only in either one of the S-genome or T-genome, it is sufficient to confirm that in the M2 obtained, no mutation has occurred in the gene on the genome that is not the target.

### [6. Breeding progeny]

One embodiment of the present invention may be the progeny of the abovementioned Nicotiana plant or of a Nicotiana plant obtained by the abovementioned production method, or may be breeding progeny obtained by crossing such Nicotiana plants. Breeding using mutants has been carried out in many plant species, including rice, wheat, barley, and soybean. For example, in the case of a mutant isolated from a mutant population treated with a mutagen, many mutations other than the mutation in the target gene are also present. Therefore, in general, backcrossing is performed to eliminate unnecessary mutations. At this time, by crossing with a cultivated variety having desirable traits, the trait carried by the mutant can be introduced into the cultivated variety, thereby obtaining a cultivated variety with higher added value. Because the trait carried by the mutant is derived from the mutation, it is necessary to select individuals having the mutation in order to promote backcrossing. In this process, fewer mutations producing the intended trait (vanillin content in the present invention) leads to fewer mutations needing to be tracked, and therefore the labor required for backcrossing is reduced. In order to promote efficient backcrossing, there is a need for a method for simply detecting the presence or absence of the mutation, and whether the mutation is homozygous or heterozygous. This method can be carried out using the mutation detection methods to be described later. In addition, by performing MAS (Marker Assisted Selection) using background markers that show polymorphism between the mutant and the cultivated variety, lines with a high rate of return to the cultivated variety can be efficiently obtained with fewer crosses. In the case of Nicotiana plants, known SNPs and SSRs (Simple Sequence Repeats) can be used as polymorphic markers. If necessary, by sequencing the genome of the Nicotiana used, differences in nucleotide sequences or differences in repeat numbers can be identified, allowing new polymorphic markers to be acquired and used.

### [7. Other matters]

An embodiment of the present invention provides a method for determining a Nicotiana plant with an increased vanillin content. The determination method comprises the following steps:
a step of obtaining a sample by collecting a portion of a Nicotiana plant;
a step of detecting, in the genome contained in the sample, a mutation that specifically induces functional suppression of the abovementioned endogenous CAD gene; and
a step of determining a Nicotiana plant in which the mutation was detected as a Nicotiana plant having an increased vanillin content.

Here, the functional suppression results in an increase in vanillin content in leaf tobacco or dried leaves of the Nicotiana plant. That is, the abovementioned determination method is used in methods for producing Nicotiana plants, etc.

One embodiment of the present invention provides a method for breeding Nicotiana plants. The method comprises a step of crossing Nicotiana plants having a high vanillin content, as determined by the abovementioned determination method.

One embodiment of the present invention provides the progeny of the abovementioned Nicotiana plant, of a Nicotiana plant obtained by the abovementioned production method, of a Nicotiana plant determined by the abovementioned determination method, or of a Nicotiana plant obtained by the abovementioned breeding method, or breeding progeny obtained by crossing such Nicotiana plants. For example, when the Nicotiana plant is a Nicotiana plant, and is also Nicotiana tabacum, an increased vanillin content is produced by functional suppression of the two CAD genes (NtCAD-T and NtCAD-S). Therefore, breeding becomes possible based on a two-factor recessive inheritance pattern, using the mutations on the NtCAD-T gene and on the NtCAD-S gene as markers, and the labor involved in breeding is reduced in comparison with conventional methods.

Breeding using mutants has been carried out in many plant species. For example, a mutant isolated from a mutant population treated using a mutagen has many mutations other than the mutation in the gene of interest. Therefore, in general, backcrossing is performed to eliminate unnecessary mutations. In this crossing, by crossing the mutant with a cultivated variety possessing desirable traits, the desired trait carried by the mutant can be introduced into an existing cultivated variety. The breeding progeny obtained in this manner can be varieties that impart high added value to existing cultivated varieties. In this process, fewer target mutations producing an increase in vanillin content leads to fewer mutations needing to be tracked, and therefore the labor required for backcrossing is reduced. The abovementioned Nicotiana plant can produce an increase in vanillin content through a mutation in a single gene, and can therefore be suitably used from the standpoint of breeding.

The present invention is not limited to the embodiments described above, and various modifications can be made within the scope disclosed in the claims, and embodiments obtained by appropriately combining the technical means disclosed in the different embodiments are also included within the technical scope of the present invention.

### (Summary)

The present invention may be summarized below by combining the embodiments described above

(1) A tobacco material comprising a portion of a Nicotiana plant, wherein
   the Nicotiana plant has a mutation accompanied by functional suppression of an endogenous cinnamyl alcohol dehydrogenase gene in at least one of:
      (a) an endogenous cinnamyl alcohol dehydrogenase gene comprising, as a coding region, a polynucleotide encoding a polypeptide having at least 90% sequence identity to the amino acid sequence shown in SEQ ID NO: 1; and
      (b) an endogenous cinnamyl alcohol dehydrogenase gene comprising, as a coding region, a polynucleotide encoding a polypeptide having at least 90% sequence identity to the amino acid sequence shown in SEQ ID NO: 2, and
   the tobacco material contains 0.05 ppm or more of vanillin.
(2) The tobacco material as disclosed in (1), wherein the tobacco material comprises a dried tobacco material.
(3) The tobacco material as disclosed in (1) or (2), wherein the tobacco material is in the form of any of a cut filler, a powder, particles, a sheet, granules, or an extract.
(4) A tobacco product comprising the tobacco material as disclosed in any one of (1) to (3).
(5) The tobacco product as disclosed in (4), which is any of an electronic tobacco product, a cigarette, and a smokeless tobacco product.
(6) A Nicotiana plant, wherein a mutation inducing functional suppression of an endogenous cinnamyl alcohol dehydrogenase gene is introduced into at least one of:
   (a) an endogenous cinnamyl alcohol dehydrogenase gene comprising, as a coding region, a polynucleotide encoding a polypeptide having at least 90% sequence identity to the amino acid sequence shown in SEQ ID NO: 1; and
   (b) an endogenous cinnamyl alcohol dehydrogenase gene comprising, as a coding region, a polynucleotide encoding a polypeptide having at least 90% sequence identity to the amino acid sequence shown in SEQ ID NO: 2, and
   a vanillin content of a harvested material obtained from the abovementioned Nicotiana plant is higher than a vanillin content of a harvested material obtained from the same amount of a wild-type Nicotiana plant.
(7) The Nicotiana plant as disclosed in (6), belonging to Nicotiana tabacum or Nicotiana rustica.
(8) The Nicotiana plant as disclosed in (6) or (7), wherein the vanillin content is at least twice the vanillin content of leaves harvested from the same amount of a wild-type Nicotiana plant.
(9) The Nicotiana plant as disclosed in any of (6) to (8), wherein the mutation is introduced by means of mutagen treatment, genome editing, or gene knockout.
(10) The Nicotiana plant as disclosed in any of (6) to (9), wherein the mutation is introduced into a coding region of at least one of said endogenous cinnamyl alcohol dehydrogenase genes.

### EXAMPLES

Examples of the present invention will be described below.

### [Example 1: Obtaining tobacco CAD mutant]

The genome sequence of the tobacco (Nicotiana tabacum) cultivar "Tsukuba 1" was analyzed, and CAD considered to be derived from the S-genome (NtCAD-S, ID: nttv1s022g02367, SEQ ID NO: 5) and CAD considered to be derived from the T-genome (NtCAD-T, ID: nttv1s791g11064, SEQ ID NO: 6) were identified. The CDS sequences of NtCAD-S (SEQ ID NO: 3) and NtCAD-T (SEQ ID NO: 4) were subjected to homology searches against the NCBI database, confirming that the genomic sequences shown in SEQ ID NO: 5 and 6 are sequences each relating to the CAD genes of tobacco (Nicotiana tabacum). The amino acid sequence of NtCAD-S (SEQ ID NO: 1) and the amino acid sequence of NtCAD-T (SEQ ID NO: 2) were subjected to a search in the same way, confirming that the amino acid sequences shown in SEQ ID NO: 1 and 2 are each amino acid sequences of CAD.

Tobacco mutants having mutations in CAD-S or CAD-T were obtained. The nucleotide sequences of the NtCAD-S and NtCAD-T gene regions were analyzed in 2000 tobacco mutant lines, and mutations were identified. Specifically, seeds of the selfed progeny of the mutant (M2 seeds) obtained from each M1 generation of 2000 tobacco mutants produced by EMS treatment of seeds of the tobacco cultivar Tsukuba 1 were sown, and DNA extracted from eight seedlings per line was pooled (Tajima et al. (2011) Ann. Phytopathol. Soc. Jpn. 77: 258), and the nucleotide sequences were analyzed. As a result, three lines with nonsense mutations in the coding region of the NtCAD-S gene (SEQ ID NO: 3) and two lines with nonsense mutations in the coding region of NtCAD-T (SEQ ID NO: 4) were identified. Two mutants were selected for each gene. For NtCAD-S, the following lines were selected: a line in which the C at position 2237 from the 5' end of the genomic sequence shown in SEQ ID NO: 5 was mutated to a T, that is, a line in which the C at position 112 from the 5' end of the CDS sequence shown in SEQ ID NO: 3 was mutated to a T (NtCAD-S-1, SEQ ID NO: 7), and a line in which the C at position 2282 from the 5' end was mutated to a T, that is, a line in which the C at position 157 from the 5' end of the CDS sequence shown in SEQ ID NO: 3 was mutated to a T (NtCAD-S-2, SEQ ID NO: 8); and for NtCAD-T, the following lines were selected: a line in which the C at position 3833 from the 5' end of the genomic sequence shown in SEQ ID NO: 6 was mutated to a T, that is, a line in which the C at position 394 from the 5' end of the CDS sequence shown in SEQ ID NO: 4 was mutated to a T (NtCAD-T-1, SEQ ID NO: 9), and a line in which the C at position 4725 from the 5' end was mutated to a T, that is, a line in which the C at position 715 from the 5' end of the CDS sequence shown in SEQ ID NO: 4 was mutated to a T (NtCAD-T-2, SEQ ID NO: 10). Seeds of these lines were sown, and DNA was extracted from seedlings. Using this DNA as a template, PCR was performed with primers to identify individuals homozygous for the mutations. KOD One (registered trademark) PCR Master Mix (TOYOBO) was used for PCR. As a result, two lines carrying mutations in the NtCAD-S gene and two lines carrying mutations in the NtCAD-T gene were obtained. Table 1 below lists the line names, the CAD nucleotide sequences of the lines, and the primer sequences used for mutation confirmation or mutant selection.

**[Table 1]**

| System name | Nucleotide SEQ ID NO: | Primer sequence |
|---|---|---|
| NtCAD-S-1 | SEQ ID NO: 7 | Mutant selection primer |
| | | SEQ ID NO: 11 (Forward) |
| | | SEQ ID NO: 12 (Reverse) |
| | | Mutation confirmation primer |
| | | SEQ ID NO: 19 (Forward) |
| | | SEQ ID NO: 20 (Reverse) |
| NtCAD-S-2 | SEQ ID NO: 8 | Mutant selection primer |
| | | SEQ ID NO: 13 (Forward) |
| | | SEQ ID NO: 14 (Reverse) |
| | | Mutation confirmation primer |
| | | SEQ ID NO: 19 (Forward) |
| | | SEQ ID NO: 20 (Reverse) |
| NtCAD-T-1 | SEQ ID NO: 9 | Common primer for mutant selection and mutation confirmation |
| | | SEQ ID NO: 15 (Forward) |
| | | SEQ ID NO: 16 (Reverse) |
| NtCAD-T-2 | SEQ ID NO: 10 | Common primer for mutant selection and mutation confirmation |
| | | SEQ ID NO: 17 (Forward) |
| | | SEQ ID NO: 18 (Reverse) |

NtCAD-S-1 and NtCAD-T-1 were grown in a greenhouse and crossed to obtain an F1 generation (NtCAD-F1-1). Furthermore, NtCAD-S-2 and NtCAD-T-2 were grown in a greenhouse and crossed to obtain an F1 generation (NtCAD-F1-2). Each F1 generation was grown in a greenhouse and selfed to obtain two F2 generations derived from different mutant combinations (NtCAD-F2-1 and NtCAD-F2-2). These two F2 generations were sown, DNA was extracted from the transplanted seedlings, and PCR was then performed using the primers listed in Table 1 to amplify the surrounding sequences including the mutations. PCR was once again performed on the amplified product to add P7 and P5 sequences and individual barcodes for sequencing with iSeq 100 (Illumina), and sequencing analysis was conducted to determine the genotype of each individual. As a result, individuals homozygous for mutations in both NtCAD-S and NtCAD-T were identified: NtCAD-sstt-A (hereinafter "CAD-A") and NtCAD-sstt-B (hereinafter "CAD-B"). Individuals lacking mutations in both genes were also identified: NtCAD-SSTT-A (a sister line of NtCAD-sstt-A) and NtCAD-SSTT-B (a sister line of NtCAD-sstt-B). These individuals were selfed to obtain the F3 generation.

### [Example 2: Evaluation of smoking taste-contributing components]

### (Samples)

Eighteen individuals each of the F3 generations of CAD-A and CAD-B were grown in the field, and topping was performed at the flowering stage. Seven weeks after topping, three mature leaves were collected from each individual. As a control, Tsukuba 1 was grown in the same manner, and three mature leaves were collected. Leaf collection was performed from 15 individuals in good condition (12 individuals for CAD-A), and leaves from three individuals (nine leaves in total) were mixed in equal proportions to prepare one sample, yielding five samples in total (four samples for CAD-A only). The harvested leaves were subjected to yellowing and curing, and the cured leaves were used as cured leaf samples. From each sample, three leaves were selected, the midribs were removed, and the leaves were freeze-dried to obtain mature leaf samples.

From leaves harvested from three plants of each line of the Nicotiana plants, the midribs were collected and used as leaf vein samples. The leaf vein samples of the CAD-A mutant and the CAD-B mutant were mixed in equal proportions and then pulverized. Hereinafter, samples obtained by mixing and pulverizing multiple samples are collectively referred to simply as "CAD".

For the CAD-A mutant at the flowering stage (immediately after topping), the stems harvested after leaf collection, and the stems of the control Tsukuba 1 harvested at the same stage, had the epidermis peeled to expose the cortex and then forced-dried at 60°C for 48 hours for use as xylem-after-topping samples.

Seven weeks after topping, stems were harvested from two plants each of the CAD-A mutant, the CAD-B mutant, and the control Tsukuba 1 after leaf collection, the stems had the epidermis peeled to expose the cortex and then forced-dried at 60°C for 48 hours to obtain xylem-after-harvest samples. The samples of the CAD-A mutant and the CAD-B mutant were mixed in equal proportions and then pulverized.

### (Results)

Three of the five cured leaf samples, and one sample each of the mature leaf sample, the xylem-after-harvest sample, the xylem-after-topping sample, and the leaf vein sample, were pulverized and subjected to component analysis by GC-MS and LC-MS/MS. The results of the GC-MS and LC-MS/MS analyses are shown in Tables 2, 3, and 4. For the cured leaf samples only, the tables show the mean values (n = 3) and the significance of differences from the control Tsukuba 1 (n = 3). Statistical analysis was performed using Dunnett's test (**p < 0.01, *p < 0.05). In both CAD mutants, CAD-A and CAD-B, vanillin showed significant differences from the control in all sample types, namely mature leaf samples, cured leaf samples, xylem-after-harvest samples, xylem-after-topping samples, and leaf vein samples, and the vanillin content was significantly increased in the CAD mutants. Specifically, compared with the control, the CAD mutants showed: 2.2-6.4-fold higher vanillin content in mature leaf samples (CAD-A, CAD-B); 1.3-4.3-fold higher content in cured leaf samples (CAD-A, CAD-B); 9-fold higher content in xylem-after-harvest samples (CAD); 25-fold higher content in xylem-after-topping samples (CAD-A); and 4.4-fold higher content in leaf vein samples (CAD).

Table 2 shows the semi-quantitative values of vanillin and other components in the cured leaf samples, xylem-after-harvest samples, xylem-after-topping samples, and leaf vein samples.

**[Table 2]**

| Table 2. Semi-quantitative values of components in cured leaf samples, xylem-after-harvest samples, xylem-after-topping samples, and leaf vein samples (relative values against Tsukuba 1) | | | | | | |
|---|---|---|---|---|---|---|
| | | Cured Leaf | | Stalk without epidermis | | Leaf Vein |
| | | | | After Topping | After harvest | |
| | Tsukuba 1 | CAD-A | CAD-B | CAD-A | CAD | CAD |
| Vanillic acid | 1 | 2.81 ** | 4.82 ** | 1.88 | 1.10 | 2.33 |
| Coniferyl alcohol | 1 | 1.73 ** | 2.41 ** | 3.59 | 2.68 | 2.35 |
| Vanillin | 1 | 1.29 | 4.34 ** | 25.14 | 9.05 | 4.43 |
| Vanillyl alcohol | 1 | 4.17 ** | 5.23 ** | 3.95 | 2.87 | 2.94 |
| Sinapaldehyde | 1 | 0.00 ** | 0.00 ** | 0.91 | 0.56 | N.D. |
| Sinapyl alcohol | 1 | 0.64 * | 0.97 | 0.87 | 0.89 | 1.64 |
| phenethylamine | 1 | 0.86 | 5.52 ** | N.D. | N.D. | 4.34 |
| Vanillin glucoside | 1 | 4.72 ** | 5.71 ** | 5.21 | 1.26 | 3.35 |
| Coniferin | 1 | 0.92 | 0.91 | 3.75 | 0.46 | 1.00 |
| Syringin | 1 | 0.71 | 1.66 | N.D. | 9.15 | 0.73 |
| Chlorogenic acid | 1 | 0.90 | 0.91 | 0.18 | 0.01 | 1.53 |
| Scopolin | 1 | 1.70 ** | 2.56 ** | 1.17 | 0.04 | 4.78 |
| Vanillylamine | N.D. | detection | detection | detection | detection | detection |
| Caffeic acid | 1 | 1.29 ** | 0.59 ** | 0.12 | 0.01 | 1.29 |
| Scopoletin | 1 | 2.10 ** | 4.76 ** | 5.01 | 4.18 | 3.08 |
| p-Coumaric acid | 1 | 0.54 ** | 1.40 ** | 7.47 | 0.96 | 0.76 |
| Sinapinic acid | 1 | 2.68 ** | 3.09 ** | N.D. | N.D. | 2.68 |
| Ferulic acid | 1 | 2.34 ** | 1.95 ** | N.D. | N.D. | 3.73 |
| Cinnamic acid | 1 | 0.45 | 3.03 ** | N.D. | N.D. | 1.35 |

| | | | | | | |
|---|---|---|---|---|---|---|
| * : Significance level 5% or less ** : Significance level 1% or less | | | | | | |

Table 3 shows the semi-quantitative values of vanillin and other components in the mature leaf samples.

**[Table 3]**

| Table 3. Semi-quantitative values of components in mature leaf samples relative values against Tsukuba 1) | | | |
|---|---|---|---|
| | Leaf | | |
| | Tsukuba 1 | CAD-A | CAD-B |
| Vanillic acid | 1 | 1.45 | 2.07 |
| Coniferyl alcohol | 1 | 1.92 | 2.61 |
| Ferulic acid | 1 | 5.90 | 9.31 |
| Vanillin | 1 | 2.18 | 6.39 |
| Vanillyl alcohol | 1 | 4.28 | 5.13 |
| Scopoletin | 1 | 2.76 | 5.48 |
| Sinapaldehyde | 1 | 0.77 | 0.74 |
| Sinapyl alcohol | 1 | 0.90 | 1.17 |
| phenethylamine | 1 | 0.69 | 3.29 |
| Vanillin glucoside | 1 | 1.75 | 1.48 |
| Coniferin | 1 | 0.46 | 0.36 |
| Syringin | 1 | 0.45 | 0.41 |
| Chlorogenic acid | 1 | 0.96 | 1.12 |
| Scopolin | 1 | 0.93 | 0.74 |
| Vanillylamine | N.D. | detection | detection |
| Caffeic acid | 1 | 1.07 | 1.08 |
| Scopoletin | 1 | 6.65 | 1.88 |
| p-Coumaric acid | N.D. | N.D. | N.D. |
| Sinapinic acid | N.D. | N.D. | N.D. |
| Ferulic acid | N.D. | N.D. | N.D. |
| Cinnamic acid | N.D. | N.D. | N.D. |

Table 4 shows the quantitative values of vanillin in the cured leaf samples, xylem-after-topping samples, and leaf vein samples of the CAD mutants.

**[Table 4]**

| Table 4. Quantitative values of vanillin in cured leaf samples, xylem-after-topping samples, and leaf vein samples | |
|---|---|
| | Vanillin content of cured samples (ppm) |
| Xylem-after-topping sample (CAD-A) | 200 |
| Cured leaf sample (CAD-B) | 40 |
| Leaf vane sample (CAD) | 15 |

### [Example 3: Preparation of tobacco materials and smoking taste evaluation]

Using the xylem-after-topping material of the CAD-A mutant and the cured leaves of Tsukuba 1, tobacco materials were prepared, applied to cigarettes, and subjected to a smoking taste test.

The cured leaves of Tsukuba 1 were pulverized and then prepared so that the ratio of cured leaf:propylene glycol (hereinafter, PG) was 1:3, thereby producing the tobacco material. Meanwhile, the method for preparing the tobacco material from the xylem-after-topping of the CAD-A mutant was as follows: using food-additive-grade 95% alcohol (Traceable 95), extraction was performed at a solid-liquid ratio of 1:9 for approximately 1 hour at 30-40°C, after which the solid and liquid phases were separated, the ethanol fraction was concentrated to dryness, and the residue was dried and pulverized with grading. Finally, the dried concentrate and the residue powder were mixed with PG so that the ratio with PG was 1:4. Non-flavored cigarettes were prepared, and the tobacco materials produced from the cured leaves of Tsukuba 1 and from the xylem-after-topping of the CAD-A mutant were applied to the cut filler.

Compared with the cured leaves of the control Tsukuba 1, five of six trained panelists evaluated the xylem-after-topping material of the CAD-A mutant as having increased sweetness, and all six of six panelists evaluated it as having a vanilla-like aroma. These results demonstrate that the stems of the CAD mutants provide enhanced smoking taste characteristics.

### [Example 4: Acquisition of tobacco CAD single mutants, and evaluation of smoking taste-contributing components]

For the two F2 generations obtained in Example 1 (NtCAD-F2-1 and NtCAD-F2-2), sequencing analysis was performed to determine the genotype of each individual, and individuals homozygous for a mutation in either the NtCAD-S gene or the NtCAD-T gene were identified as follows: NtCAD-ssTT-A (hereinafter, "CAD-A-sT"), NtCAD-SStt-A (hereinafter, "CAD-A-St"), NtCAD-ssTT-B (hereinafter, "CAD-B-sT"), and NtCAD-SStt-B (hereinafter, "CAD-B-St"). These individuals were selfed to obtain the F3 generation.

Eighteen individuals of each of the F3 generations of CAD-A-sT, CAD-A-St, CAD-B-sT, and CAD-B-St were grown in the field, and topping was performed at the flowering stage. Seven weeks after topping, three mature leaves were collected from each individual. As a control, Tsukuba 1 was grown in the same manner, and three mature leaves were collected. Leaf collection was performed from 15 individuals in good condition, and leaves from three individuals (nine leaves in total) were mixed in equal proportions to prepare one sample, yielding five samples in total. The harvested leaves were subjected to yellowing and curing, and the cured leaves were used as cured leaf samples. From each sample, three leaves were selected and pulverized, and the samples were subjected to component analysis by GC-MS and LC-MS/MS.

GC-MS analysis was specifically performed as follows. 0.05 g of the pulverized cured leaf sample was placed into a 15-mL screw-cap glass centrifuge tube, and 50 µL of an internal standard solution (20 ppm 6-methylquinoline) was added to each weighed sample. 10 mL ethanol was added, the mixture was stirred with a vortex mixer, and the tube was tightly capped. Shaking extraction was performed using a medium-sized thermostatic shaking incubator, Bioshaker (registered trademark) BR-43FH/MR (Taitec Corporation, Saitama) at 200 rpm, 50°C, for 1 hour. The extract was filtered through a 0.45-µm PTFE filter and subjected to GC-MS analysis. Other detailed analytical conditions are as follows.
- GC/MS: 7890B/5977B (Agilent Technologies, Tokyo)
- Column: DB-WAX UI; 122-7032UI; 0.25 mm × 30 m × 0.25 µm (Agilent Technologies, Tokyo)
- Injection mode: Splitless
- Inlet temperature: 250°C
- Oven program: 50°C for 2 min; 20°C/min to 120°C for 2 min; 10°C/min to 250°C for 24 min
- Measurement time: 44.5 min
- Post run: 5.0 mL/min; 30 min
- Measurement mode: SIM/SCAN

LC-MS/MS analysis was specifically performed as follows. 0.1 g of the pulverized cured leaf sample was placed into a 15-mL screw-cap glass centrifuge tube, and 5.0 mL of methanol solution containing phlorizin (internal standard) at 2.0 µg/mL was added, the tube was capped with a screw cap, and extraction was performed using a Bioshaker (registered trademark) BR-43FH/MR (Taitec Corporation, Saitama) at 250 rpm, 70°C, for 1 hour. The extract was filtered through a 0.45-µm PTFE filter and subjected to liquid chromatography-tandem mass spectrometry (LC-MS/MS) analysis. Other detailed analytical conditions are as follows.
- High-speed liquid chromatograph: 1260 Infinity (Agilent Technologies, Tokyo)
- Mass spectrometer: 6470 Triple Quad LC/MS (Agilent Technologies, Tokyo)
- Column: InfinityLab Poroshell 120 CS-C18, internal diameter 2.1 mm, length 150 mm, particle size 2.7 µm (Agilent Technologies, Tokyo)
- Column temperature: 35°C
- Eluent A: 20 mM ammonium formate-ultrapure water
- Eluent B: 20 mM ammonium formate-acetonitrile:ultrapure water = 9:1 (v/v)
- Elution conditions:
   1) 0-2 minutes: Eluent A:eluent B = 95:5 flow for 2 minutes (eluent A 95%/eluent B 5%)
   2) 2-10 minutes: Apply linear concentration gradient from eluent A:eluent B = 95:5 to 55:45
   3) 10-20 minutes: Apply linear concentration gradient from eluent A:eluent B = 55:45 to 0:100
   4) 20-25 minutes: Eluent B flow for 5 minutes (eluent A 0%/eluent B 100%)
- Flow velocity: 0.2 mL/min
- Injection volume: 2.0 µL
- Ion source parameters:
   Ionization method: electrospray ionization (ESI)
   Nebulizer gas: nitrogen
   Nebulizer gas temperature: 350°C
   Nebulizer gas flow rate: 5 L/min
   Nebulizer pressure: 35 psi
   Sheath gas: 350°C
   Sheath gas flow rate: 11 L/min
   Capillary voltage: 3500 V
- Mass spectrometer parameters:
   Ion polarity: negative
   Collision gas: nitrogen
   Measurement mode: selected reaction monitoring (SRM)

### (Results)

In all four CAD single-mutant lines of CAD-A and CAD-B, vanillin showed no significant difference compared with the control Tsukuba 1. Table 5 shows the semi-quantitative values of vanillin and other components in the cured leaf samples of the CAD single-mutant individuals obtained as a result of the GC-MS and LC-MS/MS component analysis.

**[Table 5]**

| Table 5: Semi-quantitative values of components in cured leaf samples of CAD single-mutant individuals (relative values against Tsukuba 1) | | | | | |
|---|---|---|---|---|---|
| | Cured Leaf | | | | |
| | Tsukuba 1 | CAD-A | | CAD-B | |
| Mutant type of CAD gene | ST | sT | St | sT | St |
| Vanillic acid | 1 | 1.04 | 0.98 | 1.30 | 1.49 |
| Conifer alcohol | 1 | 0.78 | 0.90 | 0.88 | 0.91 |
| Vanillin | 1 | 0.71 | 0.73 | 0.84 | 1.38 |
| Vanillyl alcohol | N.D. | N.D. | N.D. | N.D. | N.D. |
| Sinapyl aldehyde | 1 | 0.70 | 0.81 | 0.80 | 0.92 |
| Sinapyl alcohol | 1 | 0.95 | 0.95 | 2.15 | 1.14 |
| phenethylamine | 1 | 1.42 | 2.74 | 5.41 | 1.75 |
| Vanillin glucoside | 1 | 0.70 | 1.12 | 0.72 | 0.96 |
| Coniferin | 1 | 1.43 | N.D. | 2.30 | 1.41 |
| Syringin | 1 | 1.55 | 0.73 | 3.46 | 1.01 |
| Chrologenic acid | 1 | 0.83 | 1.01 | 1.02 | 1.11 |
| Scopolin | 1 | 1.21 | 0.98 | 2.05 | 2.07 |
| Vanillyl amine | 1 | 0.68 | 0.99 | 0.95 | N.D. |
| Caffeic acid | 1 | 0.65 | 0.70 | 0.31 | 1.03 |
| Scop oletin | 1 | 0.94 | 1.00 | 2.80 | 2.46 |
| p-Coumaric acid | 1 | 1.22 | 1.15 | 1.53 | 0.80 |
| Sinapic acid | 1 | 1.15 | 1.33 | 1.91 | 1.82 |
| Ferulic acid | 1 | 0.64 | 1.01 | 0.85 | 2.07 |
| p-Cinnamic acid | 1 | 1.03 | 1.17 | 1.23 | 1.21 |

### [Example 5: Evaluation of smoking taste-contributing components in stem tissue]

### (Analysis of vanillin and vanillin analogs in stem tissue)

The F3 generations of Tsukuba 1, CAD-A, and CAD-B were grown in the field, and stems were harvested at the flowering stage. Stems from nine plants of each line were harvested and then immediately separated into three tissues, epidermis, cortex, and pith, and each tissue was dried at 60°C for 48 hours. The dried materials obtained from each tissue were finely pulverized using a Lab Mill LM-05 (Dalton Corporation) and subjected to component analysis by GC-MS and LC-MS/MS.

The results of the GC-MS and LC-MS/MS analyses are shown in Table 6. In Table 6, the values are expressed as relative values, with the semi-quantitative value of each tissue (epidermis, cortex, pith) of the control Tsukuba 1 as 1. In both CAD-A and CAD-B mutants, all analyzed components except vanillylamine showed an increasing trend. In particular, in the cortex, vanillin increased 7.97- to 11.77-fold in both CAD-A and CAD-B mutants, and ferulic acid increased 11.17- to 24.46-fold compared with the control Tsukuba 1. Furthermore, quantitative analysis of vanillin in each tissue of Tsukuba 1 and CAD-A showed that, in the cortex, Tsukuba 1 contained 12.00 ppm, whereas CAD-A contained 108.90 ppm, and of the total vanillin in the stem, 60-77.45% was contained in the cortex. In addition, in the cortex of both CAD-A and CAD-B mutants, vanillin glucoside, which was not detected in the cortex of Tsukuba 1, was detected. Meanwhile, in the pith of both CAD-A and CAD-B mutants, vanillylamine was not detected.

**[Table 6]**

| | Tsukuba 1 | | | CAD-A | | | CAD-B | | |
|---|---|---|---|---|---|---|---|---|---|
| | Epidermis | Cortex | Pith | Epidermis | Cortex | Pith | Epidermis | Cortex | Pith |
| Vanilln | 1 | 1 | 1 | 2.20 | 11.77 | 10.03 | 2.38 | 7.97 | 8.21 |
| Vanillic acid | 1 | 1 | 1 | 5.72 | 1.91 | 2.41 | 3.16 | 3.42 | 5.60 |
| Vanillil alcohol | 1 | 1 | 1 | 3.60 | 3.93 | 3.79 | 3.93 | 6.39 | 4.73 |
| Vanillyl amine | N.D. | 1 | 1 | N.D. | 0.69 | N.D. | N.D. | 0.63 | N.D. |
| Ferulic acid | 1 | 1 | 1 | 1.38 | 11.17 | 3.60 | 2.13 | 24.46 | 5.49 |
| Vanillin glucoside | 1 | N.D. | 1 | 9.84 | Detected | 13.25 | 1.53 | Detected | 6.89 |

### (Analysis of vanillin content by stem region and harvest stage)

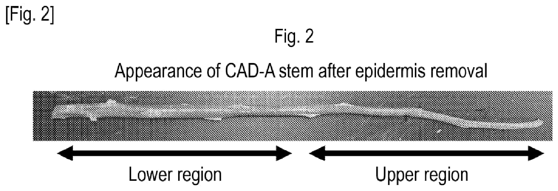

The F3 generation of CAD-A was grown in the field, and stems were harvested at both the flowering stage and the harvest stage. Stems from nine individuals were harvested and then immediately stripped of the epidermis and divided into two regions, upper and lower, cut from the central portion of the stem (see fig. 2), and each region was dried at 60°C for 48 hours. The dried materials from the nine individuals obtained were combined into samples by mixing material from three individuals to form one level, yielding three levels in total, which were then finely pulverized using a Lab Mill LM-05 (Dalton Corporation) and subjected to component analysis by GC-MS.

The results of the GC-MS analysis are shown in Table 7. For CAD-A stems at the flowering stage (without epidermis), vanillin in the lower region was 6.85-fold higher than in the upper region, vanillic acid was 1.66-fold higher, and vanillyl alcohol was 1.73-fold higher. Meanwhile, for CAD-A stems at the harvest stage (without epidermis), vanillin in the lower region was 1.47-fold higher than in the upper region, vanillic acid was 1.24-fold higher, and vanillyl alcohol was 0.73-fold.

**[Table 7]**

| | CAD A stem (without epidermis) | | | |
|---|---|---|---|---|
| | Flowering stage | | Harvest stage | |
| | Lower region | Upper region | Lower region | Upper region |
| Vanillin | 6.85 | 1 | 1.47 | 1 |
| Vanillic acid | 1.66 | 1 | 1.24 | 1 |
| Vanillyl alcohol | 1.73 | 1 | 0.86 | 1 |

### [Example 6: Preparation of tobacco materials by fine pulverization treatment, high-temperature/high-pressure treatment, steam distillation treatment, and ethanol extraction treatment, and evaluation of smoking taste-contributing components]

Using the xylem-after-topping (without epidermis), cured leaves and leaf veins of CAD-A and Tsukuba 1, various treatments were performed to prepare tobacco materials. Each CAD-A and Tsukuba 1 sample used was finely pulverized using a Lab Mill LM-05 (Dalton Corporation), and after applying the treatments A-D described below, or without treatment (untreated), 50 mg of each tobacco material was collected, and the vanillin content in each tobacco material was quantitatively analyzed by GC-MS.

### (A: Fine pulverization treatment)

For the cured leaves of Tsukuba 1 and CAD-A, tobacco materials were prepared by mixing the cured leaves with propylene glycol (hereinafter, PG) so that the ratio of cured leaf:PG was 1:3. For the leaf veins, tobacco materials were prepared by the same procedure, except that the cured leaves were replaced with leaf veins. For the xylem-after-topping (without epidermis), components were first extracted using 99.5% ethanol at a solid-liquid ratio of 1:9 for approximately 1 hour at 30-40°C, after which the solid and liquid phases were separated, the ethanol fraction was concentrated to dryness, and the residue was dried and pulverized. Finally, the dried concentrate and residue powder were mixed with PG so that the ratio with PG was 1:4, and the mixture was again finely pulverized using a laboratory mixer L5M-A (Silverson) to prepare the tobacco material.

### (B: High-temperature/high-pressure treatment)

Using the xylem-after-topping (without epidermis) of Tsukuba 1 and CAD-A, mixtures were prepared so that the ratio of xylem-after-topping (without epidermis):water was 1:7, and high-temperature/high-pressure treatment was performed in a TAS-01 reaction vessel (Taiatsu Techno Corp.) under conditions of a temperature of 150°C and a pressure of 380 kPa, for 3 hours with stirring, thereby producing the tobacco material. For the leaf veins, tobacco materials were prepared by the same procedure, except that the xylem-after-topping (without epidermis) described above was replaced with leaf veins.

### (C: Steam distillation treatment)

Using 400 g of xylem-after-topping (without epidermis) of Tsukuba 1 and CAD-A, tobacco materials were prepared. A cylindrical pot was filled with 4 L of tap water and brought to a boil at 100°C using an IH heater. After boiling, heating was temporarily stopped, and 400 g of the xylem-after-topping (without epidermis) sample was added to the pot. The lid of the cylindrical pot was closed, heating was resumed, and the distillation operation was carried out for 2 hours. A condenser was connected to tap water, and tap water was passed therethrough to function as a chiller. The distillation treatment was performed for 100 minutes, 1.0 L of the resulting steam-distilled sample was collected, and sodium chloride was added to 20% (w/w). 1 L of ethyl acetate was added to the steam-distilled sample, and liquid-liquid transfer was performed using a separating funnel. The ethyl acetate layer was collected and concentrated under reduced pressure using a rotary evaporator to obtain a dried material. The essential oil obtained was approximately 465 mg/kg for CAD-A and approximately 335 mg/kg for Tsukuba 1. For quantitative analysis of the vanillin content, the essential oil was diluted 100-fold with 99.5% ethanol and used for testing.

### (D: Ethanol extraction treatment)

Using the xylem-after-topping (without epidermis) of Tsukuba 1 and CAD-A, mixtures were prepared so that the ratio of xylem-after-topping (without epidermis):99.5% ethanol was 1:5, and the mixtures were left to stand at 25°C for 24 hours. After 24 hours, the supernatant was collected to prepare the tobacco material.

### (Quantitative analysis of vanillin content in each tobacco material)

Into each 15-mL screw-cap test tube, 10.0 mL of an ethanol solution containing the internal standard 6-methylquinoline at a concentration of 0.1 µg/mL was added, followed by addition of 50 mg of each tobacco material prepared by abovementioned treatments A-D, or untreated tobacco material. The tubes were capped with a screw cap, and shaking extraction was performed using a medium-sized thermostatic shaking incubator, Bioshaker (registered trademark) BR-43FH/MR (Taitec Corporation, Saitama) at 200 rpm, 50°C, for 1 hour. The extract was filtered through a 0.45-µm PTFE filter and subjected to GC-MS analysis. Other detailed analytical conditions are as follows.
- Gas chromatograph analysis apparatus: 7890B (Agilent Technologies, Tokyo)
- Column: DB-WAX UI, internal diameter 0.25 mm, length 30 m, particle size 0.25 µm (Agilent Technologies, Tokyo)
- Sample inlet temperature: 250°C
- Column oven temperature-increase conditions
   1) 0-2 minutes: hold at 50°C
   2) 2-7.5 minutes: increase temperature to 120°C at 20°C/min, then hold for 2 minutes
   3) 7.5-50.5 minutes: increase temperature to 250°C at 10°C/min, then hold for 30 minutes
- Carrier gas: helium
- Flow velocity: 1.0 mL/min
- Sample injection volume: 1.0 µL
- Sample injection method: pulsed splitless (20 psi, 2 min)
- Mass spectrometer: 5977A MSD (Agilent Technologies, Tokyo)
- Ionization method: Electron impact ionization (EI)
- Ion polarity: positive
- Electron energy: 70 eV
- Mass ion source temperature: 230°C
- Mass quadrupole temperature: 150°C

The quantitative and qualitative ions for the internal standard 6-methylquinoline were set as shown in Table 8.

**[Table 8]**

| Table 8: Quantification of vanillin, 6-methylquinoline, and 1,3-dimethoxybenzene, and qualifying ions | | | | |
|---|---|---|---|---|
| Compound name | Retention time (min) | Molecular weight | Quantifying ion (m/z) | Qualifying ion (m/z) |
| Vanillin | 19.2 | 152.2 | 151.0 | 152.0 |
| 6-methylquinoline (internal standard for vanillin quantification) | 14.7 | 143.2 | 143.0 | 142.0 |
| 1,3-dimethylbenzene (internal standard for quantification of vanillin after alkali heating treatment) | 11.5 | 138.2 | 138.0 | 109.0 |

The quantitative value of vanillin was calculated by the calibration-curve method, using the ratio of the area value of the vanillin ion chromatogram at m/z = 151.0 to the area value of the internal standard 6-methylquinoline ion chromatogram at m/z = 143.0. Calibration solutions, which were prepared by adding the internal standard 6-methylquinoline at a concentration of 100 ng/mL to vanillin standard solutions in the concentration range of 7.81-250 ng/mL, at four concentration levels (7.81, 62.5, 125, 250 ng/mL) were analyzed, and the calibration curve showing linearity with a correlation coefficient of 0.9999 for the area ratio of the ion chromatograms of 6-methylquinoline and vanillin was used for quantification.

### (Results)

The vanillin content (ppm) per 1 g of each quantified sample is shown in Table 9. In the untreated samples, the vanillin content of the control Tsukuba 1 was 9.67 ppm in the xylem-after-topping (without epidermis), 9.98 ppm in the cured leaf, and 5.17 ppm in the leaf vein, whereas in untreated CAD-A, the vanillin content was 80.11 ppm in the xylem-after-topping (without epidermis), 21.32 ppm in the cured leaf, and 7.01 ppm in the leaf vein. In the fine pulverization treatment, the vanillin content of the control Tsukuba 1 was 4.18 ppm in the xylem-after-topping (without epidermis), 9.98 ppm in the cured leaf, and 3.18 ppm in the leaf vein, whereas in CAD-A, the vanillin content was 33.07 ppm in the xylem-after-topping (without epidermis), 4.77 ppm in the cured leaf, and 3.34 ppm in the leaf vein. In the high-temperature/high-pressure treatment, the vanillin content of the control Tsukuba 1 was 14.27 ppm in the xylem-after-topping (without epidermis), whereas in CAD-A, the vanillin content was 56.84 ppm in the xylem-after-topping (without epidermis) and 7.66 ppm in the leaf vein. In the steam distillation treatment, the vanillin content of the control Tsukuba 1 in the xylem-after-topping (without epidermis) was 30.53 ppm in the essential-oil stock solution, whereas in CAD-A, the vanillin content in the xylem-after-topping (without epidermis) was 995.75 ppm in the essential-oil stock solution. In the ethanol extraction treatment, the vanillin content of the control Tsukuba 1 in the xylem-after-topping (without epidermis) was 2.93 ppm, whereas in CAD-A, the vanillin content in the xylem-after-topping (without epidermis) was 17.02 ppm. In addition, the external aroma of the ethanol extract of the CAD-A xylem-after-topping (without epidermis) exhibited a vanilla-like aroma.

**[Table 9]**

| | | | In 1 g sample |
|---|---|---|---|
| Variety | Tissue | Treatment | Vanillin content (ppm) |
| Tsukuba 1 | Xylem-after-topping (without epidermis) | None | 9.67 |
| | Cured leaf | None | 9.98 |
| | Leaf vein | None | 5.17 |
| | Xylem-after-topping (without epidermis) | Fine pulverization | 4.18 |
| | Cured leaf | Fine pulverization | 4.54 |
| | Leaf vein | Fine pulverization | 3.18 |
| | Xylem-after-topping (without epidermis) | High-temperature/ high-pressure | 14.27 |
| | Xylem-after-topping (without epidermis) | Steam distillation | 30.53 |
| | Xylem-after-topping (without epidermis) | Ethanol extraction | 2.93 |
| CAD-A | Xylem-after-topping (without epidermis) | None | 80.11 |
| | Cured leaf | None | 21.32 |
| | Leaf vein | None | 7.01 |
| | Xylem-after-topping (without epidermis) | Fine pulverization | 33.07 |
| | Cured leaf | Fine pulverization | 4.77 |
| | Leaf vein | Fine pulverization | 3.34 |
| | Xylem-after-topping (without epidermis) | High-temperature/ high-pressure | 56.84 |
| | Leaf vein | High-temperature/ high-pressure | 7.66 |
| | Xylem-after-topping (without epidermis) | Steam distillation | 995.75 |
| | Xylem-after-topping (without epidermis) | Ethanol extraction | 17.02 |

### [Example 7: Preparation of tobacco materials subjected to alkali heating treatment, and evaluation of smoking taste-contributing components and smoking taste]

Using the xylem-after-topping (without epidermis), cured leaves and leaf veins of CAD-A and Tsukuba 1, an alkali heating treatment was performed to prepare tobacco materials. Each CAD-A and Tsukuba 1 sample used was finely pulverized using a Lab Mill LM-05, and after applying the treatment described below, 50 mg of each tobacco material was collected, and the vanillin content in each tobacco material was quantitatively analyzed by GC-MS.

### (Alkali heating treatment)

The alkali heating treatment method was as follows.
1) 3.0 g of each finely pulverized sample was added to 40 mL of 1M NaOH, and a heating extraction treatment was performed at 80°C for 2 hours under atmospheric pressure.
2) The mixture was neutralized with 1M phosphoric acid solution.
3) The liquid phase was filtered, ethyl acetate was mixed with the filtrate, and the fraction eluted into the ethyl acetate (ethyl acetate extract) was distilled to obtain a dried material. The dried material was re-dissolved in ethanol
4) The residue was dried at 60°C for 2 hours.
5) The ethanol re-dissolved material of the dried material obtained in 3) was returned onto the residue from 4), the ethanol was air-dried, and the resulting material was used as the tobacco material for alkali heating treatment.

As a control, in 1) above, 40 mL of 1M NaOH was replaced with 40 mL of distilled water, and 2) was omitted; the tobacco material prepared in this manner was designated as Mock-treated tobacco material.

### (Quantitative analysis of vanillin content in Mock-treated tobacco material)

Into each 15-mL screw-cap test tube, 10.0 mL of an ethanol solution containing the internal standard 6-methylquinoline at a concentration of 0.1 µg/mL was added, followed by addition of 50 mg of tobacco material prepared by the Mock treatment described above. The tubes were capped with a screw cap, and shaking extraction was performed using a medium-sized thermostatic shaking incubator, Bioshaker (registered trademark) BR-43FH/MR (manufactured by Taitec Corporation) at 200 rpm, 50°C, for 1 hour. The extract was filtered through a 0.45-µm PTFE filter and subjected to GC-MS analysis.

### (Method for extracting vanillin in the alkali heating treatment)

Into a 15-mL screw-cap test tube, 30.0 µL of an acetone solution of the internal standard 1,3-dimethoxybenzene (50 µg/mL) was added, followed by addition of 100 mg of the pulverized tobacco material prepared by the alkali heating treatment described above, and 3.0 mL of 1M NaOH in Milli-Q water was added. The tube was capped with a screw cap and heated at 80°C for 1 hour using a heat block. After heating, the test tube was allowed to cool to room temperature, and 210 µL of 85% phosphoric acid aqueous solution was introduced into the test tube and vortexed to neutralize. After stirring, 3.0 mL of ethyl acetate was added and vortexed, and vanillin and 1,3-dimethoxybenzene were transferred into the organic layer. A centrifuge tube was centrifuged at 2000 rpm for 3 minutes to separate the material into two layers, and the upper (organic) layer was subjected to GC-MS analysis.

Except for the items described above, the remaining detailed analytical conditions for the GC-MS analysis of the Mock-treated and alkali heating-treated samples were identical.
- Gas chromatograph analysis apparatus: 7890B (Agilent Technologies, Tokyo)
- Column: DB-WAX UI, internal diameter 0.25 mm, length 30 m, particle size 0.25 µm (Agilent Technologies, Tokyo)
- Sample inlet temperature: 250°C
- Column oven temperature-increase conditions
   1) 0-2 minutes: hold at 50°C
   2) 2-7.5 minutes: increase temperature to 120°C at 20°C/min, then hold for 2 minutes
   3) 7.5-50.5 minutes: increase temperature to 250°C at 10°C/min, then hold for 30 minutes
- Carrier gas: helium
- Flow velocity: 1.0 mL/min
- Sample injection volume: 1.0 µL
- Sample injection method: pulsed splitless (20 psi, 2 min)
- Mass spectrometer: 5977A MSD (Agilent Technologies, Tokyo)
- Ionization method: Electron impact ionization (EI)
- Ion polarity: positive
- Electron energy: 70 eV
- Mass ion source temperature: 230°C
- Mass quadrupole temperature: 150°C

The quantitative and qualitative ions for vanillin, and for the internal standards used for quantifying vanillin in the Mock treatment and alkali heating treatment, namely 6-methylquinoline and 1,3-dimethoxybenzene, were set as shown in Table 10.

**[Table 10]**

| Table 10: Quantification of vanillin, 6-methylquinoline, and 1,3-dimethoxybenzene, and qualifying ions | | | | |
|---|---|---|---|---|
| Compound name | Retention time (min) | Molecular weight | Quantifying ion (m/z) | Qualifying ion (m/z) |
| Vanillin | 19.2 | 152.2 | 151.0 | 152.0 |
| 6-methylquinoline (internal standard for vanillin quantification) | 14.7 | 143.2 | 143.0 | 142.0 |
| 1,3-dimethylbenzene (internal standard for quantification of alkali heating-treated vanillin) | 11.5 | 138.2 | 138.0 | 109.0 |

The quantitative value of the Mock-treated vanillin was calculated by the calibration-curve method, using the ratio of the area value of the vanillin ion chromatogram at m/z = 151.0 to the area value of the internal standard 6-methylquinoline ion chromatogram at m/z = 143.0. Calibration solutions, which were prepared by adding the internal standard 6-methylquinoline at a concentration of 100 ng/mL to vanillin standard solutions in the concentration range of 7.81-250 ng/mL, at four concentration levels (7.81, 62.5, 125, 250 ng/mL) were analyzed by GC-MS, and the calibration curve showing linearity with a correlation coefficient of 0.9999 for the area ratio of the ion chromatograms of 6-methylquinoline and vanillin was used for quantification.

The quantitative value of alkali heating-treated vanillin was calculated by the calibration-curve method, using the ratio of the area value of the vanillin ion chromatogram at m/z = 151.0 to the area value of the internal standard 1,3-dimethoxybenzene ion chromatogram at m/z = 138.0. Vanillin standard solutions in the concentration range of 3.13-100 µg/mL, to which the internal standard 1,3-dimethoxybenzene had been added at a concentration of 50 µg/mL, were prepared at six concentration levels (3.13, 6.25, 12.5, 25.0, 50, 100 µg/mL) as calibration solutions, and 30 µL of each was placed into a screw-cap test tube, and the procedure described in the method for extracting cell-wall-bound vanillin was carried out to obtain an ethyl acetate solution, which was analyzed by GC-MS, and a calibration curve showing linearity with a correlation coefficient of 0.9999 for the area ratios of the ion chromatograms of 1,3-dimethoxybenzene and vanillin was used for quantification.

### (Vanillin content quantification results)

The vanillin content of the tobacco materials subjected to alkali heating treatment was 60.56 ppm for the xylem-after-topping (without epidermis) of the control Tsukuba 1, 45.35 ppm for the xylem-after-topping (with epidermis), and 15.69 ppm for the cured leaf, while for CAD-A the vanillin content was 1491.59 ppm for the xylem-after-topping (without epidermis), 504.62 ppm for the xylem-after-topping (with epidermis), and 74.27 ppm for the cured leaf. For CAD-B, the vanillin content in the cured leaf subjected to alkali heating treatment was 79.94 ppm. In the xylem-after-topping (without epidermis), the vanillin content in CAD-A increased 24.6-fold compared with the control Tsukuba 1.

### (Smoking taste evaluation of tobacco materials)

Using the tobacco materials prepared from the xylem-after-topping of Tsukuba 1 and CAD-A, both untreated and subjected to alkali heating treatment, smoking taste evaluations were conducted in both cigarette form and heated-tobacco form. For the cigarette smoking taste evaluation, the total weight of cut filler packed into each cigarette was set at 715 mg, and each tobacco material was blended so that its weight corresponded to 5% of the weight of the unflavored cigarette cut filler. Specifically, 35 mg of each tobacco material was blended per cigarette evaluated. For the heated-tobacco smoking taste evaluation, reconstituted sheets were prepared using the tobacco materials by means of the method described below, and were mixed with unflavored papermaking-method reconstituted tobacco cut filler. The total weight of tobacco material packed into the heated portion of the heated-tobacco product was set at 254 mg, and the reconstituted sheet prepared from each tobacco material sample was blended so that its weight corresponded to 15% of the weight of the unflavored papermaking-method reconstituted tobacco cut filler. Specifically, 54 mg of reconstituted sheet was blended per article evaluated.

### (Preparation of reconstituted sheets)

Reconstituted sheets for smoking taste evaluation in heated-tobacco products were prepared by the following procedure.
1) A slurry was prepared by mixing 3 g of tobacco material, 20 mL of water, and 1.8 g of carboxymethyl cellulose, followed by further addition of 1.8 g of glycerol.
2) A horizontal glass-plate base was wrapped with a wrap, and the slurry prepared in 1) was spread over the wrap.
3) The wrap with the spread slurry, together with the base, was placed in an oven and heated at an oven temperature of 80°C for 3 hours to dry the slurry into the form of a sheet.
4) The sheet heated and dried in 3) was left to cool and cut into pieces of 2 mm × 10 mm.

### (Smoking taste evaluation in cigarettes)

Five trained panelists conducted a cigarette smoking taste evaluation. For cigarettes blended with the alkali heating-treated tobacco material of Tsukuba 1, one of the five panelists reported an increase in sweetness compared with cigarettes blended with untreated tobacco material of the control Tsukuba 1. For cigarettes blended with untreated CAD-A tobacco material, all five panelists reported an increase in sweetness compared with the control. For cigarettes blended with the alkali heating-treated CAD-A tobacco material, all five panelists reported a stronger increase in sweetness compared with the control, and additionally reported perceiving a vanilla-like aroma.

### (Smoking taste evaluation in heated tobacco)

Five trained panelists conducted a smoking taste evaluation for heated tobacco. For heated tobacco blended with the alkali heating-treated tobacco material of Tsukuba 1, one of the five panelists reported an increase in sweetness compared with heated tobacco blended with untreated tobacco material of the control Tsukuba 1. For heated tobacco blended with untreated CAD-A tobacco material, four of the five panelists reported an increase in sweetness compared with the control. For heated tobacco blended with the alkali heating-treated CAD-A tobacco material, four of the five panelists reported an increase in sweetness compared with the control, and three of the five panelists additionally reported perceiving a vanilla-like aroma.

### INDUSTRIAL APPLICABILITY

The present invention can be utilized for tobacco products.

### [Sequence Listing]

International Application based on the Patent Cooperation Treaty JT24494 Tobacco material, tobacco product, and P24046123 20241226-00120072252402854294regular20241226105729202408281653385600_P1AP101__JT_10.x ml

## Claims

1. A tobacco material comprising a portion of a Nicotiana plant, wherein
the Nicotiana plant has a mutation accompanied by functional suppression of an endogenous cinnamyl alcohol dehydrogenase gene in at least one of:
(a) an endogenous cinnamyl alcohol dehydrogenase gene comprising, as a coding region, a polynucleotide encoding a polypeptide having at least 90% sequence identity to the amino acid sequence shown in SEQ ID NO: 1; and
(b) an endogenous cinnamyl alcohol dehydrogenase gene comprising, as a coding region, a polynucleotide encoding a polypeptide having at least 90% sequence identity to the amino acid sequence shown in SEQ ID NO: 2, and
the tobacco material contains 0.05 ppm or more of vanillin.

2. The tobacco material as claimed in claim 1, wherein the tobacco material comprises a dried tobacco material.

3. The tobacco material as claimed in claim 1 or 2, wherein the tobacco material is in the form of any of a cut filler, a powder, particles, a sheet, granules, or an extract.

4. A tobacco product comprising the tobacco material as claimed in any one of claims 1 to 3.

5. The tobacco product as claimed in claim 4, which is any of an electronic tobacco product, a cigarette, and a smokeless tobacco product.

6. A Nicotiana plant, wherein a mutation inducing functional suppression of an endogenous cinnamyl alcohol dehydrogenase gene is introduced into at least one of:
(a) an endogenous cinnamyl alcohol dehydrogenase gene comprising, as a coding region, a polynucleotide encoding a polypeptide having at least 90% sequence identity to the amino acid sequence shown in SEQ ID NO: 1; and
(b) an endogenous cinnamyl alcohol dehydrogenase gene comprising, as a coding region, a polynucleotide encoding a polypeptide having at least 90% sequence identity to the amino acid sequence shown in SEQ ID NO: 2, and
a vanillin content of a harvested material obtained from the abovementioned Nicotiana plant is higher than a vanillin content of a harvested material obtained from the same amount of a wild-type Nicotiana plant.

7. The Nicotiana plant as claimed in claim 6, belonging to Nicotiana tabacum or Nicotiana rustica.

8. The Nicotiana plant as claimed in claim 6 or 7, wherein the vanillin content is at least twice the vanillin content of a harvested material obtained from the same amount of a wild-type Nicotiana plant.

9. The Nicotiana plant as claimed in any one of claims 6 to 8, wherein the mutation is introduced by means of mutagen treatment, genome editing, or gene knockout.

10. The Nicotiana plant as claimed in any one of claims 6 to 9, wherein the mutation is introduced into a coding region of at least one of said endogenous cinnamyl alcohol dehydrogenase genes.
